# EUROPEAN PATENT APPLICATION

(11) **EP 4 480 504 A1**
(43) Date of publication of application: **25.12.2024**
(21) Application number: 23755868.9
(22) Date of filing: 17.02.2023
(51) Int. Cl.: A61L 2/14, D06F 39/00, D06F 58/20, D06F 58/32

(54) **LIGHT PLASMA MODULE AND CLOTHING PROCESSING DEVICE**

(30) Priority: 18.02.2022 CN 202210152990; 18.02.2022 CN 202210152994; 18.02.2022 CN 202210153063; 18.02.2022 CN 202210153066
(71) Applicant: Qingdao Haier Washing Machine Co., Ltd., Qingdao, Shandong 266101 (CN); Haier Smart Home Co., Ltd., Qingdao, Shandong 266101 (CN)
(72) Inventor: YU, Hanwu, Qingdao, Shandong 266101 (CN); XU, Yonghong, Qingdao, Shandong 266101 (CN); ZHANG, Lijun, Qingdao, Shandong 266101 (CN); ZHAO, Xueli, Qingdao, Shandong 266101 (CN)
(74) Representative: Beck & Rössig European Patent Attorneys
(86) International application number: PCT/CN2023/076705
(87) International publication number: WO 2023/155867

(57) **Abstract**

An optical plasma module, a clothing treatment equipment, a clothing treatment equipment with drying function, a clothing treatment equipment control method, and a clothing treatment equipment using the control method. The optical plasma module irradiates the air in the chamber of the module to form a sterilizing air, so that the chamber of the module is communicated to the clothing treatment drum through a connecting pipeline, and then controls the optical plasma generating device to input the sterilizing air into the clothing treatment equipment, so as to achieve the effect of sterilizing and deodorizing the clothes and/or the clothing treatment drum.

## Description

### TECHNICAL FIELD

The present invention belongs to the technical field of clothing treatment equipment, and in particular, relates to a clothing treatment equipment with sterilization and deodorization functions, and in particular, relates to an optical plasma module applied to the clothing treatment equipment.

### BACKGROUND

After the clothes treatment equipment runs a program, there will still be water vapor in the clothing treatment drum, which will cause bacteria to grow and produce odors. However, ventilation of the clothing treatment drum cannot completely kill the bacteria in the clothing treatment drum. In addition, the airflow during ventilation cannot remove the odor on the clothes, and even accelerates the spread of the odor inside the clothing treatment drum.

In order to solve the above problems, clothing treatment equipment usually uses ultraviolet rays, silver ions, high temperatures and ozone for sterilization. Among them, ultraviolet rays can penetrate the air to sterilize clothes, but ultraviolet rays can easily cause damage to human eyes and skin. Silver ions can inhibit the growth of mold and deodorize, but if absorbed by the human body, pathological changes may occur. High temperature treatment can inactivate bacteria, but clothes made of materials that are not resistant to high temperatures are prone to deformation. Excessive ozone can strongly irritate the human respiratory tract, causing symptoms such as sore throat, chest tightness and cough.

Optical plasma is a gas containing ions and free electrons produced by light tubes. Optical plasma and ion clusters decompose oxygen and water molecules into hydroxide, free oxygen atoms, superoxide ions and other oxidants. These molecules are extremely unstable and will decompose harmful impurities in the air into inert compounds such as carbon dioxide and water. The speed of destroying organic tissues is 180 times faster than ultraviolet light and 2000 times faster than ozone.

In view of this, the present invention is proposed.

### SUMMARY

The technical problem to be solved by the present invention is to overcome the deficiencies of the prior art and provide an optical plasma module and a clothing treatment equipment to achieve the purpose of providing the clothing treatment equipment with sterilization and deodorization functions.

In order to solve the above technical problems, the basic concept of the technical solution adopted by the present invention is:
An optical plasma module, comprising: a box body, being provided with an air inlet chamber and an air outlet chamber connected to each other. A fan is installed in the air inlet chamber to draw external air flow from an air inlet at a lower side of the air inlet chamber. An optical plasma tube is installed in the air outlet chamber, an upper part of the air outlet chamber is communicated to the air inlet chamber, and a lower part of the air outlet chamber is communicated to outside through an air outlet.

Furthermore, one end of an upper chamber of the air outlet chamber is communicated to an outlet of the fan in the air inlet chamber through a passage. A middle chamber of the air outlet chamber is equipped with the optical plasma tube horizontally inserted from one side of the middle chamber. A bottom wall of a lower chamber of the air outlet chamber is provided with the air outlet.

Furthermore, the lower chamber of the air outlet chamber is an inverted cone or an inverted frustum with a radial dimension gradually narrowing from top to bottom, and the air outlet is provided at a lowest point of the inverted cone or inverted frustum. Preferably, the lower chamber of the air outlet chamber is an inverted isosceles triangle with left and right sides gradually tilted toward a center, and the air outlet is provided at a lowest point of the inverted isosceles triangle.

Furthermore, the optical plasma tube is coaxially arranged with the air outlet chamber, and axial opposite sides of the air outlet chamber are respectively connected with the air inlet chamber through the passage for an insertion of the optical plasma tube.

Furthermore, the fan is horizontally arranged in the air inlet chamber, an inlet of the fan is centrally opened downward, an air inlet of the air inlet chamber is arranged at a bottom of the air inlet chamber and is coaxially opened opposite to the inlet of the fan. The outlet of the fan extends horizontally along a tangential direction of the fan and is connected to the upper chamber of the air outlet chamber through the passage horizontally extended. Preferably, the passage is coaxially arranged with the air outlet chamber.

Furthermore, the box body comprises a slot body and a top cover, the top cover is buckled and fixedly mounted on a top of the slot body to form the air inlet chamber and the air outlet chamber connected through the passage. The fan is mounted on the top cover, the optical plasma tube is correspondingly inserted into the air outlet chamber from one side of the slot body and fixedly mounted on the slot body. Preferably, a driving plate is mounted on an outer wall of the slot body.

The present invention also discloses a clothing treatment equipment, which comprises: a housing; and a clothing treatment drum installed in the housing; wherein, the housing is provided with any of the optical plasma module mentioned above, and the air inlet and the air outlet of the optical plasma module are respectively communicated to the clothing treatment drum through pipelines.

Furthermore, the box body of the optical plasma module is fixedly installed on the housing, and the optical plasma module is located at a lower side of a top surface of the housing and above the clothing treatment drum.

Furthermore, the housing comprises a front upper crossbeam located at a front side of a top, the box body of the optical plasma module is fixedly installed on a lower side of the front upper crossbeam; the air inlet chamber and the air outlet chamber of the optical plasma module are arranged left and right in the housing.

Furthermore, a door seal is connected between a drum opening of the clothing treatment drum and a front plate of the housing, and an air inlet j oint and an air outlet j oint are provided on the door seal, both of which can communicate the clothing treatment drum with outside. The air inlet of the optical plasma module is communicated to the air inlet joint provided on the door seal through an air inlet pipe; the air outlet of the optical plasma module is communicated to the air outlet joint provided on the door seal through an air outlet pipe.

After adopting the above technical scheme, the present invention has the following beneficial effects compared with the prior art.
1. In the present invention, the air in the chamber of the module is irradiated with optical plasma to form a sterilizing air, so that the chamber of the module is connected to the clothing treatment drum via a connecting pipe, and then the optical plasma generating device is controlled to input the sterilizing gas into the clothing treatment device, thereby achieving the effect of sterilizing and deodorizing the clothing and/or the clothing treatment drum.
2. In the present invention, the optical plasma generating device has a photocatalyst excitation layer. Under the catalysis of a specific nano-scale multiple precious metal medium, the broadband ultraviolet light tube irradiates the air, which can produce a large amount of hydroxide ions, super oxygen ions, hydrogen peroxide and pure negative oxygen ions, thereby improving the sterilization effect.
3. In the present invention, the air in the chamber of the module forms a sterilizing air under the irradiation of the optical plasma tube and is blown into the interior of the clothing treatment drum through the pipeline, which can not only sterilize the interior of the clothing treatment drum, but also sterilize the clothes in the clothing treatment drum, especially for clothes that cannot withstand high temperatures, it has a better sterilization effect.

The technical problem to be solved by the present invention is to overcome the deficiencies of the prior art, and also to provide a clothing treatment equipment to achieve the purpose of improving the sterilization efficiency of the clothing treatment equipment; another object of the present invention is to provide a clothing treatment equipment to achieve the purpose of removing odor from clothing.

In order to solve the above technical problems, the basic concept of the technical solution adopted by the present invention is:
A clothing treatment equipment comprises a clothing treatment drum; a drying air duct, an air outlet end of the drying air duct is communicated to the clothing treatment drum and is configured to supply airflow into the clothing treatment drum; wherein, an optical plasma tube is provided at the air outlet end of the drying air duct for irradiating and sterilizing the airflow flowing into the clothing treatment drum.

Furthermore, the drum opening of the clothing treatment drum is connected to the housing of the clothing treatment equipment through a door seal, a joint is provided on the door seal, and the air outlet end of the drying air duct is communicated to an inside of the clothing treatment drum through the joint provided on the door seal. The optical plasma tube is provided in the joint to perform light irradiation and sterilization on the airflow flowing into the clothing treatment drum through the joint; the optical plasma tube is installed on the door seal, and/or the air outlet end of the drying air duct, and/or the clothing treatment drum.

Furthermore, the optical plasma tube comprises a base, fixedly mounted with the door seal, with a hollow interior forming an installation chamber; an optical plasma tube core for generating sterilizing light, installed in the installation chamber of the base; a light-transmitting area is provided on the base for the sterilizing light generated by the optical plasma tube core to pass through, and the light-transmitting area is arranged toward the inside of the joint.

Furthermore, the base is columnar, and a notch is arranged on one side wall of the columnar base, the notch communicates inside and outside of the columnar base to form the light-transmitting area; the optical plasma tube core arranged inside the columnar base extends along an axis of the columnar base and at least partially overlaps with the notch.

Furthermore, a glass cover is arranged in the installation chamber of the columnar base, the glass cover is cylindrical and is arranged outside the optical plasma tube core, and the cylindrical glass cover at least covers the notch arranged on the side wall of the columnar base.

Furthermore, the columnar base of the optical plasma tube is inserted from the air inlet end of the joint and extends to the air outlet end of the joint; the optical plasma tube is arranged on one side of the joint, and the light-transmitting area arranged on one side of the columnar base of the optical plasma tube is opened toward a central axis direction of the joint.

Furthermore, an axial direction of the optical plasma tube is inclined at a certain angle to the axial direction of the j oint, an insertion end of the optical plasma tube is inclined relative to an extension end toward the side away from the joint; and the light-transmitting area provided on one side of the columnar base of the optical plasma tube is entirely within the joint.

Furthermore, one end of the columnar base of the optical plasma tube is closed and located in the joint, and an other end is open and located outside the j oint, and a fixing cap is detachably installed on the open end of the columnar base outside the j oint, and the fixing cap is configured to fix the optical plasma tube in the installation chamber inside the base.

Furthermore, a radially protruding fixing protrusion is provided on an outer periphery of the columnar base of the optical plasma tube, the fixing protrusion is provided between the light-transmitting area of the columnar base and the fixing cap, the fixing cap and the fixing protrusion are respectively located on inner and outer sides of the door seal, and are configured to fix the optical plasma tube on the door seal.

Furthermore, the fixing cap is annular, an inner circumference of the annular fixing cap is smaller than a radial dimension of the optical plasma tube core, and the optical plasma tube core and the glass cover are limited by the fixing cap and installed in the installation chamber of the columnar base.

Furthermore, a cylindrical glass cover is coaxially arranged inside the columnar base and sleeved outside the optical tube, and a first shock-absorbing gasket is clamped between one end of the cylindrical glass cover and the closed end of the base. A second shock-absorbing gasket is clamped between the other end of the cylindrical glass cover and the fixing cap of the base. Preferably, the outer periphery of the second shock-absorbing gasket is clamped between the fixing cap and the glass cover, and the inner periphery is clamped between the optical tube and the fixing cap.

After adopting the above technical scheme, the present invention has the following beneficial effects compared with the prior art.
1. In the present invention, the optical plasma tube irradiates the hot air in the drying air duct, so that hot air with sterilization and deodorization functions is formed in the drying air duct. The hot air with sterilization and deodorization functions quickly diffuses to the inside of the clothing treatment drum through the drying air duct. On the basis of ensuring the drying function of the clothing treatment equipment, the clothing is sterilized and deodorized, thereby improving the utilization efficiency of the hot air.
2. In the present invention, the optical plasma tube is arranged at the connection point between the drying air duct and the clothing treatment drum, so that the optical plasma tube directly irradiates the airflow returning into the clothing treatment drum, thereby increasing the amount of gas flowing into the clothing treatment drum after irradiation, thereby improving the sterilization and deodorization effects of the clothing treatment equipment.
3. In the present invention, the optical plasma tube has UVC ultraviolet rays and/or UVD ultraviolet rays. The UVD band ultraviolet rays can efficiently excite oxygen and water in the air to produce optical plasma groups, and the UVC band ultraviolet rays have a highly effective sterilization effect, so that the microorganisms attached to the clothing treatment drum and the clothes inside it are completely killed.

The technical problem to be solved by the present invention is to overcome the shortcomings of the prior art and provide a clothing treatment equipment to achieve the purpose of improving the sterilization efficiency of the clothing treatment equipment; another purpose of the present invention is to provide a clothing treatment equipment to achieve the purpose of removing odor from clothing.

In order to solve the above technical problems, the basic concept of the technical solution adopted by the present invention is:
A clothing treatment equipment with drying function comprises a clothing treatment drum; a clothing treatment drum; and a drying air duct for delivering airflow to the clothing treatment drum; wherein, an illumination cavity is provided in the drying air duct, and at least part of the airflow enters the clothing treatment drum through the illumination cavity; and an optical plasma tube is provided in the illumination cavity for irradiating and sterilizing the airflow flowing into the clothing treatment drum.

Furthermore, the drying air duct has a disc-shaped air outlet chamber, and a separation rib is arranged in the air outlet chamber, and the separation rib divides the disc-shaped air outlet chamber into two parts. The first part with a smaller volume constitutes the illumination cavity, and the optical plasma tube is arranged at a periphery of the illumination cavity and radially inserted into the illumination cavity.

Furthermore, an annular separation rib is provided in a middle of the disc-shaped air outlet chamber, an outer portion of the annular separation rib is provided with a first separation rib and a second separation rib arranged at an angle interval. A part between the first separation rib and the second separation rib constitutes the illumination cavity, a notch is provided on the first separation rib for introducing the airflow into the illumination cavity, and the optical plasma tube is arranged close to the second separation rib.

Furthermore, the drying air duct comprises an air inlet chamber provided with a fan, the air inlet chamber is directly communicated with the second part of the air outlet chamber through a connecting cavity extending obliquely upward. The first separation rib separates the connecting cavity from the illumination cavity, a notch is provided on the first separation rib for connecting the two cavities. At least part of the airflow flowing into the air inlet chamber flows into the illumination cavity through the notch; the notch is arranged close to a center of the annular air outlet chamber relative to the optical plasma tube.

Furthermore, a heater is provided in the second part of the annular air outlet chamber, the heater is directly arranged opposite to the air outlet end of the connecting cavity, and is configured to directly heat the airflow; the heater is arranged close to the notch on the first separation rib, and is configured to heat the airflow flowing into the illumination cavity.

Furthermore, the optical plasma tube is columnar, and an end of the columnar optical plasma tube is provided with a mounting seat located outside the drying air duct and at least partially radially protruding, and the radially protruding portion of the mounting seat is fixed to an outer wall of the drying air duct.

Furthermore, radially opposite sides of the mounting seat are respectively provided with fixing ribs protruding radially outward, the two fixing ribs are relatively fitted with the outer wall of the drying air duct, and the fixing ribs are respectively provided with through holes, screws pass through the through holes and are fixed to the drying air duct, so as to fix the mounting seat on the drying air duct.

Furthermore, a shock-absorbing gasket is provided on an outer periphery of the columnar optical plasma tube, and the shock-absorbing gasket is clamped between the fixing seat and the drying air duct.

Furthermore, a plurality of air outlets are provided on one side of the annular air outlet chamber, at least one of the air outlets is a first air outlet communicated to the illumination cavity, and other of the air outlets are second air outlets communicated to the second part; the first air outlet is opened opposite to at least part of the columnar optical plasma tube in the illumination cavity.

Furthermore, the second separation rib is provided with a bending portion protruding outward near an outer periphery of the annular air outlet chamber, and the columnar optical plasma tube is arranged in an area surrounded by the bending portion; the first air outlet is a circular opening corresponding to one side of the area surrounded by the bending portion.

After adopting the above technical scheme, the present invention has the following beneficial effects compared with the prior art.
1. In the present invention, the optical plasma tube irradiates the hot air in the drying air duct, so that hot air with sterilization and deodorization functions is formed in the drying air duct. The hot air with sterilization and deodorization functions quickly diffuses to the inside of the clothing treatment drum through the drying air duct. On the basis of ensuring the drying function of the clothing treatment equipment, the clothing is sterilized and deodorized, thereby improving the utilization efficiency of the hot air.
2. In the present invention, the optical plasma tube is arranged in an independent chamber in the drying air duct so that the optical plasma tube can illuminate part of the airflow flowing into the clothing treatment drum, thereby reducing the power of the optical plasma tube and improving the light sterilization effect on the airflow, so that the optical plasma tube's efficiency in sterilization and deodorization in the clothing treatment drum is significantly improved.
3. In the present invention, the optical plasma tube has UVC ultraviolet rays and/or UVD ultraviolet rays. The UVD band ultraviolet rays can efficiently excite oxygen and water in the air to produce optical plasma groups, and the UVC band ultraviolet rays have a highly effective sterilization effect, so that the microorganisms attached to the clothing treatment drum and the clothes inside it are completely killed.

The technical problem to be solved by the present invention is to overcome the deficiencies of the prior art, and also provide a method for controlling a clothing treatment equipment to achieve the purpose of improving the deodorization and sterilization efficiency of the clothing treatment equipment.

In order to solve the above technical problems, the basic concept of the technical solution adopted by the present invention is:
A method for controlling a clothing treatment equipment comprises: introducing air into a clothing treatment drum; obtaining a temperature inside the clothing treatment drum; determining whether the obtained temperature inside the clothing treatment drum satisfies a preset start-up condition, and if so, starting an optical plasma generating device to irradiate the incoming air flowing into the clothing treatment drum with sterilizing light.

Furthermore, before the air is introduced into the clothing treatment drum, it also includes: entering a washing and care program; starting a heating device in the air duct; controlling a rotation of the fan in the air duct to drive the air in the air duct to flow toward an air inlet of the clothing treatment drum.

Further, the determination of whether the temperature inside the clothing treatment drum obtained satisfies the preset start-up condition comprises: determining whether the temperature inside the clothing treatment drum obtained is less than or equal to a preset temperature, and if the temperature inside the clothing treatment drum obtained is less than or equal to the preset temperature, starting the optical plasma generating device.

Furthermore, the starting of the heating device in the air duct also includes: setting a target time period t for an operation of the heating device and starting timing.

Furthermore, while obtaining the temperature inside the clothing treatment drum, it also includes: determining whether the heating device has been running for a target time period t, and if so, turning off the heating device; if not, comparing the obtained temperature inside the clothing treatment drum with a preset temperature threshold interval, and controlling the heating device to be turned on or off according to the comparison result.

Furthermore, the controlling the heating device to be turned on or off according to the comparison result specifically includes: if the temperature in the clothing treatment drum obtained is within the preset temperature threshold interval, turning off the heating device; if the temperature in the clothing treatment drum obtained is greater than a maximum value of the preset temperature threshold interval, turning off the heating device; if the temperature in the clothing treatment drum obtained is less than a minimum value of the preset temperature threshold interval, maintaining operation or turning on the heating device.

Furthermore, the preset start condition is that the temperature in the clothing treatment drum is less than or equal to the maximum value of the preset temperature threshold range.

Another object of the present invention is to provide a clothing treatment equipment using any of the above-mentioned control methods, comprising: a clothing treatment drum, the clothing treatment drum is provided with an air inlet and an air outlet; an air duct, a fan and a heating device are provided in the air duct, the air outlet end of the air duct is connected with the air inlet so that the air in the air duct can flow into an interior of the clothing treatment drum; an optical plasma generating device, the optical plasma generating device is configured to irradiate the air flowing into the clothing treatment drum through the air duct.

Furthermore, a clothing feeding port is provided at a front end of the clothing treatment drum, a window pad is provided in the clothing feeding port, and a top of the window pad is connected to the air outlet end of the air duct. The optical plasma generating device includes an optical plasma tube assembly, and the optical plasma tube assembly is installed on a side of the window pad close to the air inlet or on a side of the air duct close to the air outlet end of the air duct.

Furthermore, it also includes a back plate and a back cover arranged on a rear side of the back plate, the clothing treatment drum is rotatably supported on the back plate, and the back cover and the back plate surround an air duct communicated to the air inlet; the optical plasma generating device includes the optical plasma tube assembly, and the optical plasma tube assembly is installed in the air duct.

After adopting the above technical scheme, the present invention has the following beneficial effects compared with the prior art.
1. In the present invention, the heating device heats the air, which is beneficial to the volatilization of odorous substances attached to the clothes, and determining whether to start the optical plasma generating device according to the air outlet temperature is beneficial to the strong oxidizing active substances formed after the optical plasma generating device irradiates the air to decompose the odorous substances, avoiding the high-temperature decomposition of the strong oxidizing active substances, thereby improving the deodorization and sterilization efficiency of the clothing treatment equipment.
2. In the present invention, the photo-plasma tube assembly irradiates the hot air in the air duct, so that the hot air has sterilization and deodorization functions, and then quickly diffuses into the interior of the clothing treatment drum. On the basis of ensuring the drying function of the clothing treatment equipment, the clothing is sterilized and deodorized, thereby improving the utilization efficiency of the hot air.
3. In the present invention, the optical plasma tube assembly is installed on the window pad or the air duct, so that the strong oxidizing active substances formed after the optical plasma generating device irradiates the gas quickly flow into the clothing treatment drum through the air inlet of the clothing treatment drum, thereby reducing the attenuation rate of the oxidizing active substances.

The specific implementation modes of the present invention are further described in detail below in conjunction with the accompanying drawings.

### BRIEF DESCRIPTION OF THE DRAWINGS

The accompanying drawings are part of the present invention and are used to provide a further understanding of the present invention. The exemplary embodiments of the present invention and their descriptions are used to explain the present invention, but do not constitute an improper limitation of the present invention. Obviously, the drawings described below are only some embodiments. For ordinary technicians in this field, other drawings can be obtained based on these drawings without creative work. In the accompanying drawings:
FIGS. 1 and 2 are schematic structural diagrams of a clothing treatment equipment according to one embodiment of the present invention from different viewing angles;
FIGS. 3 and 4 are schematic structural diagrams of an optical plasma module from different viewing angles in one embodiment of the present invention;
FIGS. 5 and 6 are schematic diagrams of explosion structures of an optical plasma module at different viewing angles according to one embodiment of the present invention;
FIGS. 7 and 8 are schematic structural diagrams of a clothing treatment equipment according to another embodiment of the present invention from different viewing angles;
FIG. 9 is a schematic diagram of a partial structure of a clothing treatment equipment in another embodiment of the present invention;
FIG. 10 is a schematic diagram of the A-A cross-sectional structure of FIG. 9 in another embodiment of the present invention;
FIG. 11 is an enlarged structural schematic diagram of point B in FIG. 10 in another embodiment of the present invention;
FIG. 12 is a schematic diagram of the structure of an optical plasma tube in another embodiment of the present invention;
FIG. 13 is a schematic diagram of the explosion structure of an optical plasma tube in another embodiment of the present invention;
FIG. 14 is a schematic block diagram of a clothing treatment equipment according to another embodiment of the present invention;
FIG. 15 is a schematic structural diagram of a rear drying air duct portion of a clothing treatment equipment in another embodiment of the present invention;
FIG. 16 is a schematic diagram of an exploded structure of a rear drying air duct portion of a clothing treatment equipment in another embodiment of the present invention;
FIG. 17 is a schematic cross-sectional view of the rear drying air duct portion of a clothing treatment equipment in another embodiment of the present invention;
FIG. 18 is a schematic diagram of a partial structure of a rear drying air duct portion of a clothing treatment equipment in another embodiment of the present invention;
FIG. 19 is another cross-sectional structural schematic diagram of the rear drying air duct of the clothing treatment equipment in another embodiment of the present invention;
FIG. 20 is an enlarged structural diagram of point A in FIG. 19 in another embodiment of the present invention;
FIG. 21 is a schematic flow chart of a method for controlling a clothing treatment equipment in yet another embodiment of the present invention;
FIG. 22 is a logic block diagram of a method for controlling a clothing treatment equipment in yet another embodiment of the present invention;
FIG. 23 is a schematic structural diagram of a washer-dryer in yet another embodiment of the present invention;
FIG. 24 is an exploded schematic diagram of a rear drying air duct portion of a clothes dryer in yet another embodiment of the present invention.

Description of the main components in the figure: 1. housing; 2. clothing treatment drum; 3. optical plasma tube; 4. drying air duct; 5. heater; 6. optical plasma module; 8. door seal; 9. fan; 10. control device; 60. box body; 61. air inlet chamber; 62. air outlet chamber; 621. upper chamber; 622. middle chamber; 623. lower chamber; 63. passage; 64. top cover; 65. air inlet; 66. air outlet; 67. air inlet pipe; 68. air outlet pipe; 69. slot body; 610. slot; 611. baffle; 612. convex rib; 613. retaining edge; 614. groove; 30. optical plasma tube core; 31. base; 32. glass cover; 33. fixing cap; 34. sealing pad; 35. fixing protrusion; 36. notch; 301. wiring terminal; 331. buckle; 332. clip; 80. joint; 81. passage; 82. protrusion; 83. air inlet joint; 84. air outlet joint; 101. front upper crossbeam; 102. back plate; 103. back cover; 104. gasket; 2. clothing treatment drum; 3. optical plasma tube; 321. mounting seat; 322. shock-absorbing gasket; 323. fixing rib; 324. notch groove; 325. limiting groove; 326. protruding rib; 4. drying air duct; 40. air inlet chamber; 41. connecting cavity; 42. air outlet chamber; 421. first part; 422. second part; 43. air outlet; 431. first air outlet; 432. second air outlet; 44. illumination cavity; 45. separation rib; 451. first separation rib; 452. second separation rib; 453. annular separation rib; 46. notch; 47. bending portion; 48. air inlet; 100. clothing treatment drum; 101. clothing feeding port; 200. air duct; 300. optical plasma generating device; 301. optical plasma tube assembly; 400. window pad; 500. back plate; 600. back cover; 700. fan; 800. heating device.

It should be noted that these drawings and textual descriptions are not intended to limit the conceptual scope of the present invention in any way, but rather to illustrate the concept of the present invention for those skilled in the art by referring to specific embodiments.

### DETAILED DESCRIPTION

In order to make the purpose, technical solutions and advantages of the embodiments of the present invention clearer, the technical solutions in the embodiments will be clearly and completely described below in conjunction with the drawings in the embodiments of the present invention. The following embodiments are used to illustrate the present invention but are not used to limit the scope of the present invention.

In the description of the present invention, it should be noted that the directions or positional relationships indicated by terms such as "upper", "lower", "front", "back", "left", "right", "longitudinal", "inside" and "outside" are based on the directions or positional relationships shown in the accompanying drawings, and are only for the convenience of describing the present invention and simplifying the description, rather than indicating or implying that the device or element referred to must have a specific direction, be constructed and operated in a specific direction, and therefore cannot be understood as a limitation on the present invention.

In the description of the present invention, it should be noted that, unless otherwise clearly specified and limited, the terms "installed", "connected", and "connected" should be understood in a broad sense, for example, it can be a fixed connection, a detachable connection, or an integral connection; it can be a mechanical connection or an electrical connection; it can be a direct connection or an indirect connection through an intermediate medium. For ordinary technicians in this field, the specific meanings of the above terms in the present invention can be understood according to specific circumstances.

### Embodiment 1

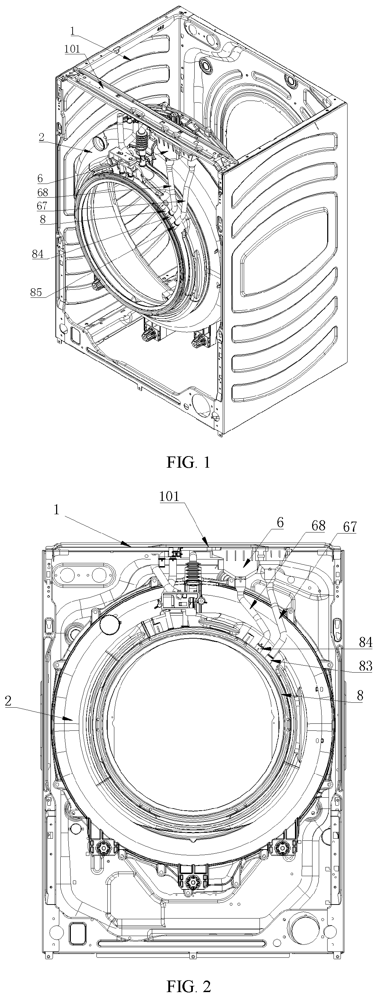

As shown in Figures 1 to 6, an optical plasma module 6 is provided in an embodiment of the present invention, which includes: a box body 60, which has an air inlet chamber 61 and an air outlet chamber 62 that are connected to each other. A fan 9 is installed in the air inlet chamber 61 to draw external air flow into the chamber from an air inlet 65 at the lower side of the chamber. An optical plasma tube 3 is installed in the air outlet chamber 62 and is horizontally inserted from one side, and the upper part of the air outlet chamber 62 is connected to the air inlet chamber 61, and the lower part is connected to the outside through the air outlet 66.

In the present invention, the air in the internal chamber of the optical plasma module 6 is formed a sterilizing gas, and the chamber of the module is connected to the clothing treatment drum 2 via a connecting pipe, and then the optical plasma module 6 is controlled to input the sterilizing gas into the clothing treatment device, thereby achieving the effect of sterilizing and deodorizing the clothing and/or the clothing treatment drum 2.

In this embodiment, the optical plasma tube 3 irradiates the gas in the internal chamber of the box body 60, so that the microorganisms carried in the gas in the chamber are completely killed, and the optical plasma tube 3 can emit optical plasma and ion clusters. The optical plasma and ion clusters emitted by the optical plasma and ion clusters decompose the oxygen and water molecules in the gas in the chamber into hydroxides, free oxygen atoms, superoxide ions and other oxidants, and decompose the odorous substances carried in the gas in the chamber into inert compounds, such as carbon dioxide and water.

In this embodiment, the gas in the chamber after being irradiated by the optical plasma tube 3 forms a sterilizing gas which is passed into the interior of the clothing treatment drum 2, which can not only sterilize the interior of the clothing treatment drum 2, but also sterilize the clothes in the clothing treatment drum 2, especially for the clothes that cannot withstand high temperature. It has a better sterilization effect, and at the same time, the odor of the clothes is quickly and easily removed.

In this embodiment, the upper chamber 621 of the air outlet chamber 62 of the optical plasma module 6 is in the shape of an elongated strip, and one end of the elongated upper chamber 621 is connected to the outlet of the fan 9 in the air inlet chamber 61 through the passage 63. The middle chamber 622 of the air outlet chamber 62 is equipped with the optical plasma tube 3 inserted horizontally from one side; the bottom wall of the lower chamber 623 of the air outlet chamber 62 is provided with an air outlet 66. By setting the air outlet chamber 62 in the shape of an elongated strip, the optical plasma tube 3 and the elongated chamber extend coaxially, and the light emitted by the optical plasma tube 3 can cover the entire area of the air outlet chamber 62, thereby improving the irradiation effect of the optical plasma tube.

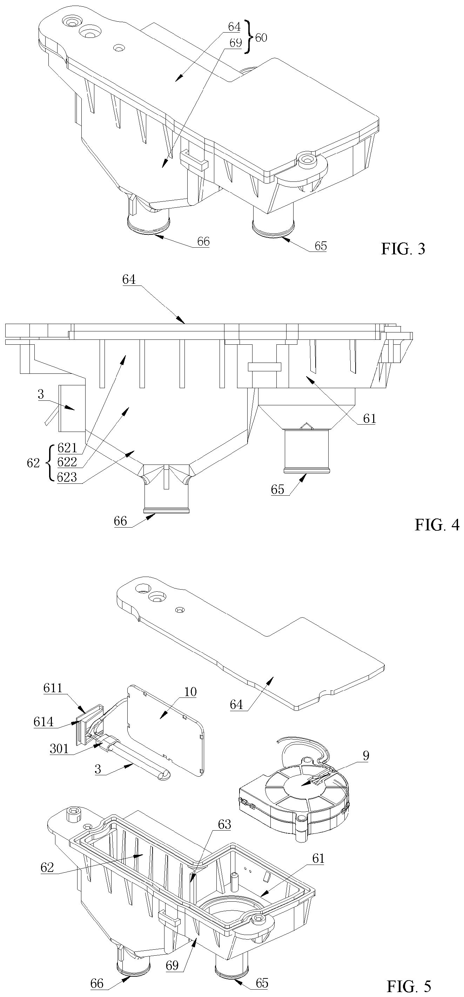

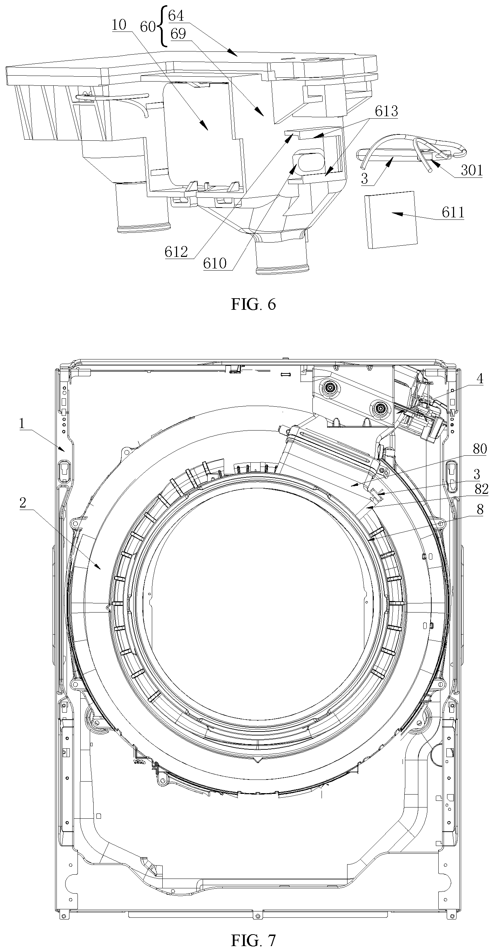

As shown in Figures 5 and 6, in this embodiment, an opening is provided on one side wall of the box body 60, which is connected to the middle chamber 622 of the air outlet chamber 62, and the optical plasma tube 3 is horizontally inserted into the air outlet chamber 62 from the opening. The optical plasma tube 3 has a wiring terminal 301 located outside the box body 60, and the radial dimension of the wiring terminal 301 is larger than the radial dimension of the opening provided on the side wall of the box body 60, so that after the optical plasma tube 3 is inserted into the air outlet chamber 62 of the box body 60 from the opening to a set position, the wiring terminal 301 abuts against the outer periphery of the opening to achieve positioning. Preferably, a slot 610 is provided on the side wall of the box body 60, and the opening is provided at the bottom of the slot 610, and the peripheral wall dimension of the slot 610 is larger than the opening dimension and is in close contact with the outer periphery of the wiring terminal 301, so as to achieve radial positioning after the optical plasma tube 3 is inserted into the slot 610.

At the same time, in this embodiment, a removable baffle 611 is also installed on the outside of the side wall of the box body 60, and the baffle 611 is abutted against the wiring terminal 301 of the optical plasma tube 3 inserted into the box body 60, so that the wiring terminal 301 of the optical plasma tube 3 is clamped between the baffle 611 and the slot 610. There is also a gap between the baffle 611 and the outer wall of the box body 60, so that the wires connected to the wiring terminal 301 can pass through the gap and be connected to the control device 10.

In this embodiment, the outer side wall of the box body 60 is provided with convex ribs 612 extending in parallel on the upper and lower sides of the opening, and the extended ends of the convex ribs 612 are respectively provided with retaining edges 613 protruding in the approaching direction, so that the two convex ribs 612 surround a slideway. The upper and lower sides of the baffle 611 are respectively provided with strip grooves 614 extending along the periphery, and the grooves 614 on the upper and lower sides of the baffle 611 are correspondingly inserted into the retaining edges 613, so that the baffle 611 can be horizontally slidably installed on the box body 60. Preferably, in order to realize the positioning of the baffle 611, the convex rib 612 is also provided on the outer side of the side wall of the box body 60 on one side of the opening, and the upper and lower ends of the convex rib 612 on the side are respectively connected to the convex rib 612 on the corresponding side, so as to surround the convex rib 612 arranged in a square shape with a notch on one side.

In the present embodiment, a side of the air outlet chamber 62 connected to the air inlet chamber 61 and the side of the air outlet chamber 62 for inserting the optical plasma tube 3 are arranged relative to each other, so that different structures are arranged at the relative positions of the box body 60, thereby improving the overall strength of the box body 60. At the same time, in the present embodiment, the end of the optical plasma tube 3 inserted into the air outlet chamber 62 is suspended and close to or directly contacts the inner wall of the air outlet chamber 62, so that the optical plasma tube 3 covers all width sections of the air outlet chamber 62, further improving the irradiation effect of the optical plasma tube 3 on the airflow. In addition, in the present embodiment, the part of the optical plasma tube 3 outside the air outlet chamber 62 is provided with a fixing seat protruding radially from the insertion hole, and the radial protrusion of the fixing seat is fixed to the outer wall of the box body 60 by screws, so as to realize the installation and fixation of the optical plasma tube and the box body.

In this embodiment, the lower chamber 623 of the air outlet chamber 62 is an inverted cone or an inverted frustum with radial dimensions gradually narrowing from top to bottom, and an air outlet 66 is provided at the lowest point of the inverted cone or the inverted frustum. Preferably, as shown in Figures 1 to 5, the lower chamber 623 of the air outlet chamber 62 is an inverted isosceles triangle with left and right sides gradually tilted toward the center, and the air outlet 66 is provided at the lowest point of the inverted isosceles triangle. Through the above arrangement, an airflow vortex is formed at the air outlet, so that the airflow in the air outlet chamber stays in the middle chamber of the air outlet chamber for a longer time, further extending the irradiation time of the optical plasma tube on the airflow passing through.

In this embodiment, the fan 9 is horizontally arranged in the air inlet chamber 61, the inlet of the fan 9 is opened in the center and downward, the air inlet 65 of the air inlet chamber 61 is arranged at the bottom of the chamber and is opened coaxially with the inlet of the fan 9. The outlet of the fan 9 extends horizontally along the tangential direction of the fan 9 and is connected to the upper chamber 621 of the air outlet chamber 62 through the horizontally extending passage 63. Thus, the centrifugal fan is installed on the module and can provide a pressurizing effect on the air flow. In addition, the air outlet 66 is a guide cone with a gradually increasing radial size from bottom to top to improve the air intake efficiency of the module.

In this embodiment, the air inlet chamber 61 is arranged higher than the optical plasma tube 3. Preferably, the air inlet 65 is arranged higher than the optical plasma tube 3 to further enhance the irradiation effect of the optical plasma tube 3 in the air outlet chamber 62. At the same time, the air inlet 65 of the optical plasma module 6 is arranged higher than the air outlet 66 to improve the air flow in the internal chamber of the module.

As shown in Figures 1 to 5, in this embodiment, the box body 60 includes a slot body 69 and a top cover 64, and the top cover 64 is buckled and fixedly installed on the top of the slot body 69 to jointly form an air inlet chamber 61 and an air outlet chamber 62 connected by a passage 63. The fan 9 is installed on the top cover 64, and the optical plasma tube 3 is inserted into the air outlet chamber 62 from one side of the slot body 69 and fixedly installed on the slot body 69.

As shown in Figures 1 to 5, this embodiment also introduces a clothing treatment equipment, which includes: a housing 1; a clothing treatment drum 2, installed in the housing 1; the housing 1 is provided with the above-mentioned optical plasma module 6, and the air inlet 65 and the air outlet 66 of the optical plasma module 6 are respectively connected to the clothing treatment drum 2 through pipelines.

In this embodiment, the box body 60 of the optical plasma module 6 is fixedly mounted on the housing 1; the optical plasma module 6 is located at the lower side of the top surface of the housing 1 and above the clothing treatment drum 2.

In this embodiment, the housing 1 includes a front upper crossbeam 101 located on the front side of the top, and the box body 60 of the optical plasma module 6 is fixedly installed on the lower side of the front upper crossbeam 101. The air inlet chamber 61 and the air outlet chamber 62 of the optical plasma module 6 are arranged on the left and right sides and are arranged in the housing 1. The air inlet 65 and the air outlet 66 of the optical plasma module 6 are both opened downward and are respectively connected to the clothing treatment drum 2 through pipelines.

In this embodiment, the clothing treatment drum 2 is arranged in the housing 1 of the clothing treatment equipment, and a door seal 8 is provided between the housing 1 and the drum mouth flange of the clothing treatment drum 2. The door seal 8 is provided with an air inlet joint 83 and an air outlet joint 84, both of which can connect the clothing treatment drum 2 with the outside; the air inlet 65 of the optical plasma module 6 is connected to the air inlet joint 83 provided on the door seal 8 via the air inlet pipe 67. The air outlet 66 of the optical plasma module 6 is connected to the air outlet joint 84 provided on the door seal 8 via the air outlet pipe 68.

In this embodiment, a valve body for controlling the opening and closing of the air inlet pipe 67 and/or the air outlet pipe 68 may be provided to control the on-off of the circulating airflow between the clothing processing tub 2 and the optical plasma module 6 (not indicated in the drawings).

In this embodiment, the door seal 8 is in a foldable and retractable cylindrical shape, and the two ends of the cylindrical door seal are sealed and connected to the barrel flange of the clothing treatment drum 2 and the front plate of the housing 1 respectively. At the same time, the drum opening provided on the barrel flange of the clothing treatment drum 2 and the clothing delivery opening provided on the front plate of the housing 1 are both inside the cylindrical door seal 8 to form a passage for taking and putting clothes. In addition, a machine door that can be turned outward to open and close the clothing delivery opening is provided on the front plate of the housing 1 of the clothing treatment device, so that the machine door can open or close the clothing treatment opening correspondingly, thereby achieving the purpose of controlling the clothing taking and putting passage formed by the above-mentioned door seal to be open and closed with the outside.

In this embodiment, the radial dimension of the air inlet pipe 67 is slightly larger than the radial dimension of the air outlet pipe 68, which is conducive to the rapid flow of the circulating airflow, so that the airflow in the air inlet pipe 67 can be quickly introduced into the chamber of the optical plasma module 6, and the optical plasma tube 3 provided in the optical plasma module 6 is used to sterilize and deodorize the airflow originating from the inside of the clothing treatment drum 2. After the airflow flows back into the clothing treatment drum, the clothes in the drum and/or the drum body can be sterilized and deodorized.

In this embodiment, the optical plasma tube 3 can be an ultraviolet light tube with at least two bands of UVC ultraviolet and UVD ultraviolet. Ultraviolet rays can be divided into vacuum ultraviolet rays (ultra -low frequency, UVD), short-wave sterilization ultraviolet rays (low frequency, UVC), medium-wave erythema effect ultraviolet rays (medium frequency, UVB), and long-wave melasma effect ultraviolet rays (high frequency, UVA) according to the wave. Among them, the UVC band of ultraviolet rays has a wavelength of 200-275nm, and the UVC band ultraviolet rays of 253.7nm have a high efficiency sterilization effect; the UVD band of ultraviolet rays has a wavelength of 100-200nm, and the UVD ultraviolet rays of 185nm can excite oxygen and water in the air to produce optical plasma groups.

In this embodiment, a control device 10 is also included, and the control device 10 is connected to the optical plasma tube 3 to control the optical plasma tube 3 to irradiate the airflow in the module; the control device 10 includes a drive board installed on the outer wall of the box body 60, and the control device 10 can be set independently or integrated on the control board of the clothing treatment equipment.

In this embodiment, an optical plasma concentration detection device is provided in the chamber of the optical plasma module 6 or the clothing treatment drum 2. The optical plasma concentration detection device is used to detect whether the optical plasma concentration in the optical plasma module or the optical plasma concentration inside the clothing treatment drum 2 into which sterilizing gas is introduced meets the sterilization requirements. The optical plasma concentration detection device is a concentration sensor (not indicated in the drawings).

In this embodiment, an odor detection device is provided inside the clothing treatment drum 2, and the odor detection device is used to detect the odor concentration and taste concentration of the clothing treatment drum 2 or the laundry inside thereof, so as to accurately control the operation of the optical plasma generating module.

In this embodiment, the optical plasma concentration detection device and the odor detection device are respectively connected to the control device 10, and the control device 10 receives the optical plasma concentration detected by the optical plasma concentration detection device and the odor concentration and taste concentration values detected by the odor detection device. The control device 10 controls the working time of the optical plasma tube 3 based on the acquired optical plasma concentration, odor concentration and taste concentration values to ensure that the optical plasma concentration in the clothing treatment drum meets the requirements of sterilization and deodorization.

Through the above-mentioned clothing treatment equipment, the optical plasma tube 3 has UVC ultraviolet rays and/or UVD ultraviolet rays. The UVD band ultraviolet rays can efficiently excite oxygen and water in the air to produce optical plasma groups, and the UVC band ultraviolet rays have a high efficiency in sterilization, so that the microorganisms attached to the clothing treatment drum 2 and the clothes therein are completely killed.

In this embodiment, the optical plasma tube 3 may also be a wide-amplitude photon tube, which emits light with balanced energy in a specific wavelength band, with a wavelength of 100nm-300nm.

In this embodiment, a photocatalytic layer is coated on the inner wall of the chamber of the optical plasma module, and a wide-amplitude photon tube is arranged on one side of the photocatalytic net, or a photocatalytic layer is provided inside the wide-amplitude photon tube, and the photocatalytic layer is composed of nano-scale precious metal catalytic materials. Under the catalysis of specific nano-scale multiple precious metal media, the wide-amplitude photon tube irradiates the air to generate a large amount of hydroxide ions, super oxygen ions, hydrogen peroxide and pure negative oxygen ions to form photohydrogen ions, which can quickly and effectively kill more than 99% of bacteria, viruses and molds in the air, and eliminate odors in the air, thereby achieving the effect of purifying the air.

In this embodiment, the wide-bandwidth photon tube can also be a broadband ultraviolet light tube. Compared with the ultraviolet light tube with UVC ultraviolet and/or UVD ultraviolet bands to irradiate the air, under the catalysis of specific nano-level multiple precious metal media, the broadband ultraviolet light tube irradiates the air, which can produce a large amount of hydroxide ions, super oxygen ions, hydrogen peroxide and pure negative oxygen ions, thereby improving the sterilization effect.

### Embodiment 2

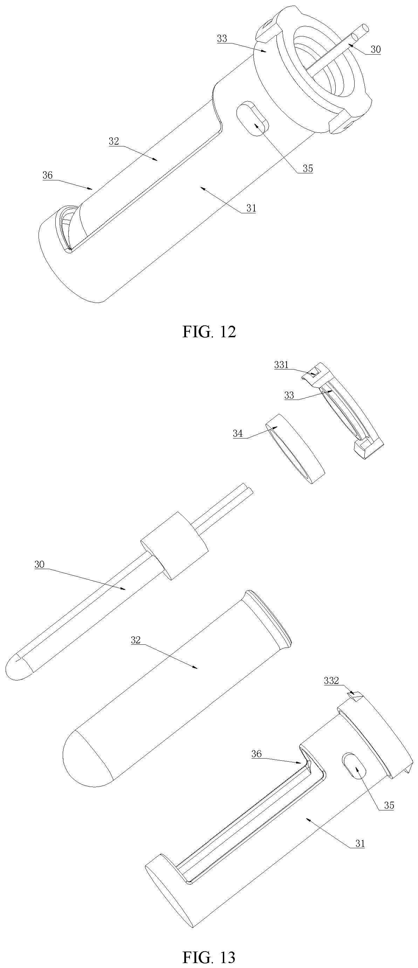

As shown in Figures 7 to 13, an embodiment of the present invention provides a clothing treatment equipment with a drying function, including a clothing treatment drum 2, and also includes a drying air duct 4 for supplying hot air into the clothing treatment drum 2, the two ends of the drying air duct 4 are respectively connected to the front and rear ends of the clothing treatment drum 2, and an optical plasma tube 3 for irradiating the hot air in the drying air duct 4 is provided in the drying air duct 4.

In this embodiment, a fan 9 is provided in the drying air duct 4, and the fan 9 is used to drive the air in the drying air duct 4 and the air inside the clothing treatment drum 2 to form a circulating airflow. A condenser is also provided in the drying air duct 4, which can be used to condense the airflow flowing through and separate the water in the circulating airflow. A heater 5 is also provided in the drying air duct 4, which can be used to heat the airflow flowing through and form a high-temperature dry airflow to flow back into the clothing treatment drum 2 to dry the clothes in the drum.

In this embodiment, after the air inside the clothing treatment drum 2 flows into the drying air duct 4, it can be irradiated by the optical plasma tube 3 arranged in the drying air duct 4, so that an airflow with sterilization and deodorization functions is formed in the drying air duct 4, and then diffused into the clothing treatment drum 2, for sterilization and deodorization of the clothes in the drum.

In this embodiment, the optical plasma tube 3 can emit optical plasma and ion clusters. The optical plasma tube 3 irradiates the hot air in the drying air duct 4. The optical plasma and ion cluster light emitted by the optical plasma tube 3 decompose the oxygen and water molecules in the air into hydroxides, free oxygen atoms, superoxide ions and other oxidants, thereby forming sterile air to sterilize the clothes, and decompose the harmful impurities in the air inside the clothing treatment drum 2 into inert compounds, such as carbon dioxide and water, so that the odor of the clothes is quickly and easily removed.

As shown in Figures 7 to 13, in this embodiment, the air outlet end of the drying air duct 4 is connected to the tube mouth of the clothing treatment drum 2, and the optical plasma tube 3 is arranged at the connecting part of the drying air duct 4 and the tube mouth of the clothing treatment drum 2 to directly irradiate the airflow returning to the clothing treatment drum, so that the sterilization and deodorization airflow formed after irradiation by the optical plasma tube 3 can directly flow back to the drum, reducing the diffusion amount of the sterilization and deodorization airflow formed by the irradiation of the optical plasma tube 3 in the drying air duct 4, thereby improving the sterilization and deodorization efficiency of the optical plasma tube.

In this embodiment, the clothing treatment equipment also includes a housing 1, and the clothing treatment drum 2 is arranged in the housing 1. A door seal 8 is provided between the housing 1 and the drum mouth flange of the clothing treatment drum 2. The air outlet end of the drying air duct 4 is connected to a joint 80 provided on the door seal 8, so that the airflow irradiated by the plasma tube 3 in the drying air duct 4 is blown into the interior of the clothing treatment drum 2 through the joint 80.

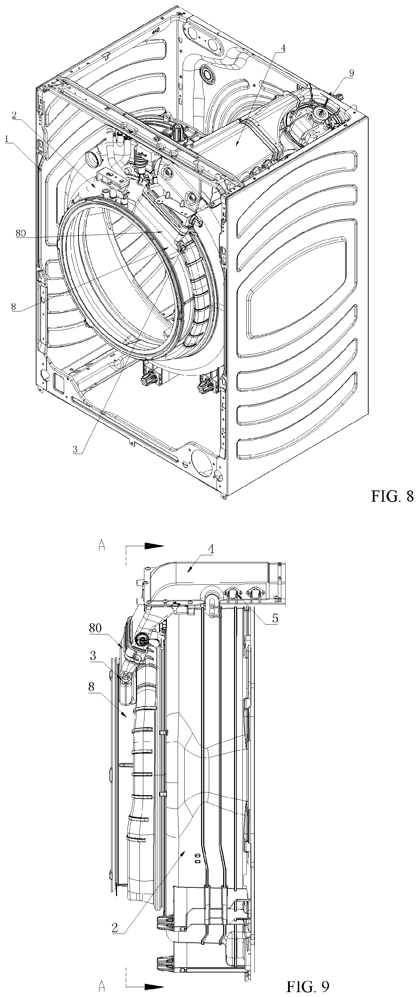

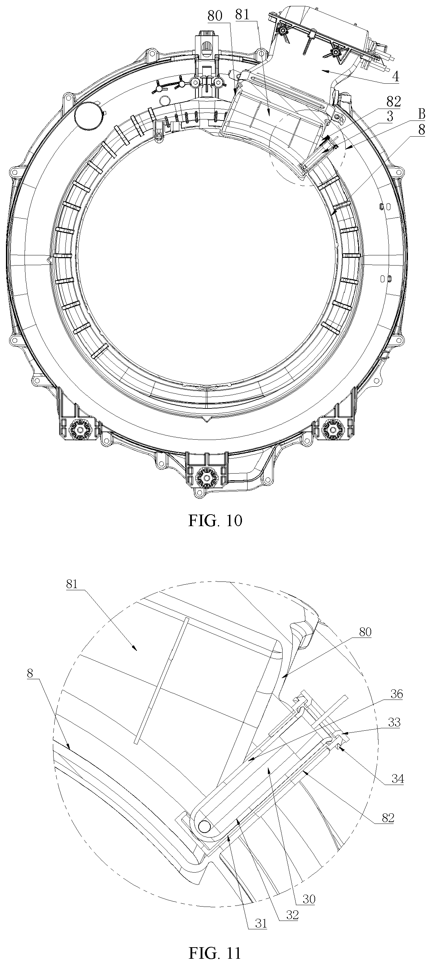

As shown in Fig. 9 and Fig. 10, in this embodiment, the door seal 8 is in the shape of a foldable and retractable cylinder, and the two ends of the cylinder door seal 8 are respectively sealed and connected to the cylinder flange of the clothing treatment drum 2 and the front plate of the housing 1. At the same time, the cylinder mouth provided on the cylinder flange of the clothing treatment drum 2 and the clothing delivery port provided on the front plate of the housing 1 are both inside the cylinder door seal 8 to form a passage for taking and putting clothes. In addition, a machine door that can be turned outward to open and close the clothing delivery port is provided on the front plate of the housing 1 of the clothing treatment device, so that the machine door can open or close the clothing treatment port accordingly, thereby achieving the purpose of controlling the clothing taking and putting passage formed by the above-mentioned door seal 8 to be open and closed with the outside.

In this embodiment, the joint 80 protrudes and extends outward from the upper part of the cylindrical door seal 8. The joint 80 can be integrally provided with the door seal 8 or separately provided with the door seal 8. Preferably, the joint 80 is integrally provided and protrudes and extends outward along the radial direction of the cylindrical door seal 8, and the extended end of the joint 80 is relatively plugged and connected with the air outlet end of the drying air duct 4.

As shown in Figures 9 to 11, in this embodiment, an opening is provided at the connection between the joint 80 and the door seal 8, which connects the internal channel of the cylindrical door seal 8 with the internal passage 81 of the joint 80; the opening is opened toward the interior direction of the clothing treatment drum 2, and is used to guide the airflow to blow into the clothing treatment drum 2.

In this embodiment, the optical plasma tube 3 is arranged in the joint 80, and the optical plasma tube 3 irradiates the airflow flowing through the joint 80 and into the clothing treatment drum 2. When the airflow flows through the joint 80 where the optical plasma tube 3 is located, the optical plasma and ion clusters emitted by the optical plasma tube 3 decompose the oxygen and water molecules in the air into hydroxides, free oxygen atoms, superoxide ions and other oxidants, and produce a certain amount of ozone; and when the airflow has a certain amount of heat, ozone is easily decomposed by heat, thereby ensuring that the optical plasma with a low ozone concentration disinfects and sterilizes the environmental space in the clothing treatment drum 2 of the clothing treatment equipment.

In this embodiment, the passage 81 inside the joint 80 is in the shape of a long strip, and the optical plasma tube 3 is disposed on one side of the long strip passage 81. The optical plasma tube 3 irradiates light toward the inside of the long strip passage 81, so that the airflow flowing through the joint 80 can be completely irradiated by light, thereby effectively improving the efficiency of sterilization and deodorization. In this embodiment, the ion clusters generated by the optical plasma tube 3 turn the particles in the air into oxidized bodies, and the harmful substances encountered, the overall photo ion clusters are very active, under the same pollution environment, it destroys the organization of organic objects 180 times faster than ultraviolet light, 2000 times faster than ozone, removes biological pollutants in the air, kills and destroys bacteria, viruses, and molds in the air and on the surface of objects, and reduces the spread of germs in the air.

In this embodiment, the fan 9 makes the air inside the drying air duct 4 and the clothing treatment drum 2 flow to form a circulating airflow. During the air flowing in the drying air duct 4, the optical plasma tube 3 irradiates the air to form sterilizing air containing oxidants to sterilize the clothing treatment equipment.

In this embodiment, a heater 5 is also provided in the drying air duct 4, and the heater 5 is arranged between the fan 9 and the optical plasma tube 3, so that after the air inside the clothing treatment drum 2 flows into the drying air duct 4, it is first heated by the heater 5 to form drying hot air, and then the drying hot air flows toward the optical plasma tube 3, which is beneficial to reduce the ozone concentration in the sterilization air.

In this embodiment, the optical plasma and ion cluster light emitted by the optical plasma tube 3 decompose the oxygen and water molecules in the air into hydroxide, free oxygen atoms, superoxide ions and other oxidants. However, along with the generation of ozone, excessive ozone will strongly irritate the human respiratory tract, causing symptoms such as sore throat, chest tightness and cough. The hot air in the drying air duct 4 decomposes the ozone by heat, which can effectively reduce the ozone concentration in the circulating air flow.

As shown in Figures 7 to 13, in this embodiment, the joint 80 is disposed from the top of the annular door seal 8 to one side, the optical plasma tube 3 is disposed at the lower side of the joint 80, and the optical plasma tube 3 irradiates light toward the internal passage 81 of the upper joint 80.

In this embodiment, a protrusion 82 protruding outward is provided on the lower side of the joint 80, and the internal space of the protrusion 82 constitutes a cavity for installing the optical plasma tube 3. The cavity is connected with the passage 81 inside the joint 80, so that the light generated by the optical plasma tube installed in the protrusion 82 can be directly irradiated into the channel and act on the airflow flowing through the channel to form a sterilization and deodorization airflow.

As shown in Figure 11 to Figure 13, in this embodiment, the optical plasma tube 3 includes:
a base 31 is fixedly mounted with the door seal 8, and the inner hollow part forms an installation chamber;
an optical plasma tube core 30 is used to generate sterilizing light and is installed in the installation chamber of the base 31;
the base 31 is provided with a light-transmitting area for the sterilizing light generated by the optical plasma tube core 30 to pass through, and the light-transmitting area is arranged toward the inside of the joint 80.

In this embodiment, the base 31 is columnar, and a notch 36 is provided on one side wall of the columnar base 31. The notch 36 connects the inside and outside of the columnar base 31 to form a light-transmitting area.

The notch 36 extends along the axis of the columnar base 31, and the radial width is the diameter of the columnar base 31.

The optical plasma tube core 30 disposed inside the columnar base 31 extends along the axis of the columnar base 31 and at least overlaps with the notch 36.

In this embodiment, a glass cover 32 is provided in the installation chamber of the columnar base 31. The glass cover 32 is cylindrical and is sleeved outside the optical plasma tube core 30. The cylindrical glass cover 32 at least covers the notch 36 provided on the side wall of the columnar base 31. The glass cover 32 is made of a transparent glass through which the light emitted by the optical plasma tube core 30 can pass, so that the light emitted by the optical plasma tube core 30 can be irradiated from the notch 36 provided on the side wall of the columnar base 31 and illuminate the airflow flowing through the passage 81 in the joint 80.

As shown in Figures 11 to 13, in this embodiment, one end of the columnar base 31 of the optical plasma tube 3 is closed and located in the joint 80, and the other end is open and located outside the joint 80. The open end of the columnar base 31 outside the joint is detachably installed with a fixing cap 33, and the fixing cap 33 is used to fix the optical plasma tube core 30 and the glass cover 32 in the installation chamber inside the base 31. In this embodiment, the fixing cap 33 is annular, and the inner radial dimension of the annular fixing cap 33 is smaller than the radial dimension of the optical plasma tube core 30, and the outer radial dimension is larger than the outer radial dimension of the columnar base 31, so that the optical plasma tube core 30 can be restricted in the internal cavity of the base 31 by the fixing cap 33. At the same time, the outer periphery of the annular fixing cap 33 is provided with two buckles 331 protruding to one side, and the outer periphery of the columnar base 31 is provided with radially protruding clips 332 corresponding to the buckle arrangement, and the buckles 331 and the clips 332 are matched for buckle connection, so that the annular fixing cap 33 can be detachably buckle-connected with the columnar base 31.

In this embodiment, a cylindrical glass cover 32 is coaxially arranged and sleeved outside the optical plasma tube core 30 in the installation chamber of the columnar base 31; the radial dimension of the cylindrical glass cover 32 is set to be equal to the radial dimension of the internal installation chamber of the columnar base 31, so that the glass cover 32 is relatively fitted and in contact with the inner wall of the base 31. At the same time, in this embodiment, a first shock-absorbing gasket is clamped between one end of the cylindrical glass cover 32 and the closed end of the base 31; a second shock-absorbing gasket is clamped between the other end of the cylindrical glass cover 32 and the fixing cap 33 of the base 31. Preferably, the outer periphery of the second shock-absorbing gasket is clamped between the fixing cap 33 and the glass cover 32, and the inner periphery is clamped between the optical plasma tube core 30 and the fixing cap 33 (not indicated in the drawings).

In this embodiment, the outer circumference of the side wall of the columnar base 31 of the optical plasma tube 3 is provided with a radially protruding fixing protrusion 35, which is provided between the light-transmitting area of the columnar base 31 and the fixing cap 33, and is used to be clamped and fixed with the through hole provided on the joint 80 of the door seal 8. In this embodiment, the radial protruding length of the fixing protrusion 35 is approximately equal to the outer circumferential size of the fixing cap 33, and both are larger than the aperture of the insertion hole on the joint 80 for inserting the columnar base 31 of the optical plasma tube 3, so that the columnar base 31 of the optical plasma tube 3 can be clamped by the fixing protrusion 35 and the fixing cap 33 on both sides of the joint 80, thereby realizing the fixed installation of the optical plasma tube 3 on the joint 80. Preferably, a sealing pad 34 made of rubber material is clamped between the columnar base 31 of the optical plasma tube 3 and the joint 80, which is used to isolate the influence of the vibration of the clothing treatment equipment on the optical plasma tube.

In this embodiment, the columnar base 31 of the optical plasma tube 3 is inserted from the air inlet end of the joint 80 and extends to the air outlet end of the joint 80. The optical plasma tube 3 is arranged on one side of the joint 80, and the light-transmitting area arranged on one side of the columnar base 34 of the optical plasma tube 3 is opened toward the central axis direction of the rectangular joint 80.

As shown in Figures 7 to 11, in this embodiment, the axial direction of the optical plasma tube 3 is inclined at a certain angle to the axial direction of the joint 80, and the insertion end of the optical plasma tube 3 is inclined relative to the extension end toward the side away from the joint 80, so that the optical plasma tube 3 and the joint 80 are staggered at a certain angle, thereby increasing the coverage area of the optical plasma tube irradiating the inside of the joint. Preferably, the axis of the optical plasma tube 3 and the axis of the joint 80 extend along different radial directions of the clothing treatment drum 2 respectively, and the extension length of the joint 80 and the optical plasma tube 3 is extended as much as possible, thereby improving the irradiation efficiency of the optical plasma tube 3 on the airflow flowing through the passage 81 in the joint 80.

At the same time, in this embodiment, the light-transmitting area provided on one side of the columnar base 31 of the optical plasma tube 3 is all located inside the joint 80, so that all the light generated by the optical plasma tube 3 can be irradiated into the passage 81 inside the joint 80, further improving the lighting effect. In addition, in this embodiment, the light-transmitting area provided on one side of the columnar base 31 of the optical plasma tube 3 has a certain extension length in the axial direction of the optical plasma tube 3. Preferably, the light-transmitting area extends from the insertion portion where the optical plasma tube 3 and the joint 80 are connected to the end.

In this embodiment, the optical plasma tube 3 may be an ultraviolet light tube having at least two wavelength bands, UVC ultraviolet and UVD ultraviolet.

Ultraviolet rays can be divided into vacuum ultraviolet rays (ultra -low frequency, UVD), short-wave sterilizing ultraviolet rays (low frequency, UVC), medium-wave erythema effect ultraviolet rays (medium frequency, UVB), and long-wave melanoma effect ultraviolet rays (high frequency, UVA) according to their wave lengths. Among them, the UVC band of ultraviolet rays has a wave length of 200-275nm, and the 253.7nm UVC band ultraviolet rays have a high efficiency in sterilization; the UVD band of ultraviolet rays has a wavelength of 100-200nm, and the 185nm UVD ultraviolet rays can excite oxygen and water in the air to produce optical plasma groups.

In this embodiment, a control device is also included, and the control device is connected to the optical plasma tube 3 to control the optical plasma tube 3 to irradiate the airflow in the drying air duct 4. The control device 10 can be independently set and installed on the housing 1, or the clothing treatment drum 2, or the drying air duct 4, or can be directly integrated on the control panel of the clothing treatment equipment.

In this embodiment, an optical plasma concentration detection device is provided in the drying air duct 4 or in the clothing treatment drum 2. The optical plasma concentration detection device is used to detect whether the optical plasma concentration in the drying air duct 4 or the optical plasma concentration inside the clothing treatment drum 2 into which sterilizing gas is introduced meets the sterilization requirements. The optical plasma concentration detection device is a concentration sensor (not shown in the accompanying drawings).

In this embodiment, an odor detection device is provided inside the clothing treatment drum 2, and the odor detection device is used to detect the odor concentration and taste concentration of the clothing treatment drum 2 or the laundry inside thereof, so as to accurately control the operation of the optical plasma generating module.

In this embodiment, the optical plasma concentration detection device and the odor detection device are respectively connected to the control device, which receives the optical plasma concentration detected by the optical plasma concentration detection device and the odor concentration and taste concentration values detected by the odor detection device. The control device controls the working time of the optical plasma tube 3 based on the acquired optical plasma concentration, odor concentration and taste concentration values to ensure that the optical plasma concentration in the drying air duct 4 meets the requirements of sterilization and deodorization.

Through the above-mentioned clothing treatment equipment, the optical plasma tube has UVC ultraviolet rays and/or UVD ultraviolet rays. The UVD band ultraviolet rays can efficiently excite oxygen and water in the air to produce optical plasma groups, and the UVC band ultraviolet rays have a highly efficient bactericidal effect, so that the microorganisms attached to the clothing treatment drum and the clothes inside it can be completely killed.

In this embodiment, the optical plasma tube 3 may also be a wide-amplitude photon tube, which emits light with balanced energy in a specific wavelength band, with a wavelength of 100nm-300nm.

In this embodiment, a photocatalytic layer is provided in the drying air duct 4, and the wide-amplitude photon tube is provided on one side of the photocatalytic net, or a photocatalytic layer is provided inside the wide-amplitude photon tube, and the photocatalytic layer is composed of nano-scale precious metal catalytic materials. Under the catalysis of specific nano-scale multiple precious metal media, the wide-amplitude photon tube irradiates the air to generate a large amount of hydroxide ions, super oxygen ions, hydrogen peroxide and pure negative oxygen ions to form photohydrogen ions, which can quickly and effectively kill more than 99% of bacteria, viruses and molds in the air, and eliminate odors in the air, thereby achieving the effect of purifying the air.

In this embodiment, the wide-bandwidth photon tube can be a broadband ultraviolet light tube. Compared with the ultraviolet light tube with UVC ultraviolet and/or UVD ultraviolet bands irradiating the air, under the catalysis of specific nano-level multiple precious metal media, the broadband ultraviolet light tube irradiates the air, which can produce a large amount of hydroxide ions, super oxygen ions, hydrogen peroxide and pure negative oxygen ions, thereby improving the sterilization effect.

### Embodiment 3

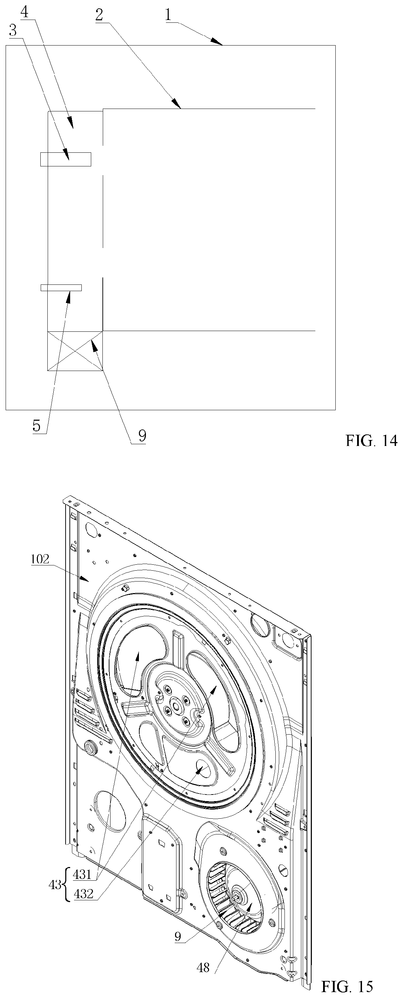

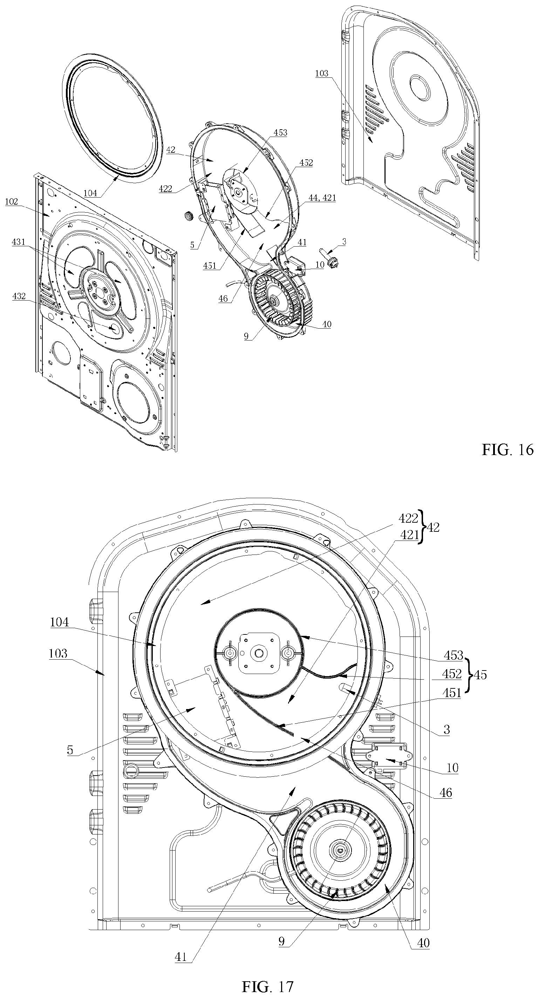

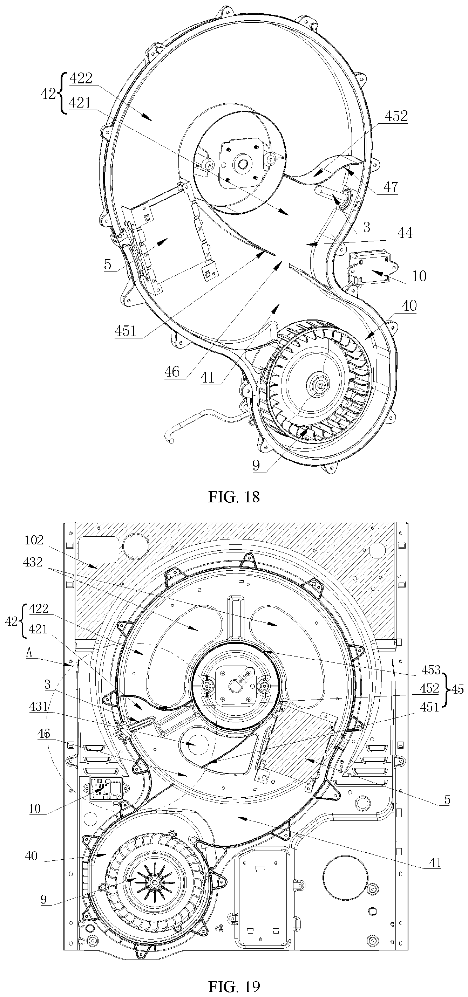

As shown in Figures 14 to 20, an embodiment of the present invention provides a clothing treatment equipment with a drying function, including a housing 1, in which a clothing treatment drum 2 is installed, and also includes a drying air duct 4 for supplying hot air into the clothing treatment drum 2, the air outlet end of the drying air duct 4 is connected to the rear portion of the clothing treatment drum 2, and the drying air duct 4 is provided with an optical plasma tube 3 for irradiating the hot air in the drying air duct 4.

In this embodiment, a fan 9 is provided in the drying air duct 4, and the fan 9 is used to drive the air flow through the drying air duct 4 to supply air to the inside of the clothing treatment drum 2. A heater 5 is also provided in the drying air duct 4, which can be used to heat the air flow passing through, form a high-temperature dry air flow to flow back into the clothing treatment drum 2, and dry the clothes in the drum. In this embodiment, the clothing treatment equipment can be a straight-discharge dryer, a circulating condensing dryer, etc.; the embodiment of the present invention takes a straight-discharge dryer as an example for explanation.

As shown in Figures 14 to 18, in this embodiment, the air inside the clothing treatment drum 2 flows into the drying air duct 4 and can be irradiated by the optical plasma tube 3 arranged in the drying air duct 4, so that an airflow with sterilization and deodorization functions is formed in the drying air duct 4, and then diffused into the clothing treatment drum 2, for sterilization and deodorization of the clothes in the drum.

In this embodiment, the optical plasma tube 3 can emit optical plasma and ion clusters. The optical plasma tube 3 irradiates the hot air in the drying air duct 4. The optical plasma and ion cluster light emitted by the optical plasma tube 3 decompose the oxygen and water molecules in the air into hydroxides, free oxygen atoms, superoxide ions and other oxidants, thereby forming sterile air to sterilize the clothes, and decompose the harmful impurities in the air inside the clothing treatment drum 2 into inert compounds, such as carbon dioxide and water, so that the odor of the clothes is quickly and easily removed.

As shown in Figures 14 to 18, in this embodiment, the optical plasma tube 3 is arranged in an illumination cavity 44 isolated from the air outlet chamber 42 of the drying air duct 4, so that the optical plasma tube 3 can illuminate part of the airflow flowing back into the clothing treatment drum 2. When the airflow flows through the illumination cavity 44 where the optical plasma tube 3 is located, the optical plasma and ion clusters emitted by the optical plasma tube 3 decompose the oxygen and water molecules in the air into hydroxides, free oxygen atoms, superoxide ions and other oxidants, and produce a certain amount of ozone. When the airflow has a certain amount of heat, ozone is easily decomposed by heat, thereby ensuring that the optical plasma with a low ozone concentration disinfects and sterilizes the environmental space in the clothing treatment drum 2 of the clothing treatment equipment.

In this embodiment, part of the air outlet chamber 42 is divided into an illumination cavity 44 by a separation rib 45, and an optical plasma tube 3 is arranged in the illumination cavity 44. An air inlet and an air outlet are arranged on opposite sides of the illumination cavity 44, and the optical plasma tube 3 is arranged near or directly at the air outlet, so that the optical plasma tube 3 irradiates light toward the inside of the illumination cavity 44, so that the airflow flowing through the illumination cavity 44 can be completely irradiated by light, thereby effectively improving the efficiency of sterilization and deodorization. In this embodiment, the ion clusters generated by the optical plasma tube 3 turn the particles in the air into oxidized bodies, and the overall light ion clusters are very active when encountering harmful substances. Under the same pollution environment, it destroys the organization of organic objects 180 times faster than ultraviolet light and 2000 times faster than ozone, removing biological pollutants in the air, killing and destroying bacteria, viruses, and molds in the air and on the surface of objects, and reducing the spread of germs in the air.

In this embodiment, the drying air duct 4 has an air inlet chamber 40, a connecting cavity 41 and an air outlet chamber 42 which are connected in sequence; a fan 9 is installed in the air inlet chamber 40, and the fan 9 draws external air into the drying air duct 4 and provides flow force to the incoming air flow.

In this embodiment, the connecting cavity of the drying air duct 4 connects the air inlet chamber 40 and the air outlet chamber 42, so that the airflow pumped by the fan 9 enters the air outlet chamber 42. The heater 5 is provided in the air outlet chamber 42, and the heater 5 heats the airflow flowing into the air outlet chamber 42 to form a high-temperature airflow, and the high-temperature airflow flows into the clothing treatment drum 2 from the rear opening of the drum. The clothes in the clothing treatment drum 2 are subjected to high-temperature drying treatment, so that the airflow flowing into the clothing treatment drum 2 is directly irradiated, so that the sterilization and deodorization airflow formed after irradiation by the optical plasma tube 3 can directly flow back into the drum. Thereby reducing the diffusion amount of the sterilization and deodorization airflow formed by the irradiation of the optical plasma tube in the drying air duct, thereby improving the sterilization and deodorization efficiency of the optical plasma tube.

In this embodiment, the fan 9 makes the air inside the drying air duct 4 and the clothing treatment drum 2 flow to form an airflow. During the air flowing in the drying air duct 4, the optical plasma tube 3 irradiates the air to form sterilizing air containing oxidants to sterilize the clothing treatment equipment.

In this embodiment, the heater 5 disposed in the drying air duct 4 is arranged close to the optical plasma tube 3, so that the heater 5 can heat the space near the optical plasma tube 3, which is beneficial to reduce the ozone concentration in the sterilizing air.

In this embodiment, the optical plasma and ion cluster light emitted by the optical plasma tube 3 decompose the oxygen and water molecules in the air into hydroxide, free oxygen atoms, superoxide ions and other oxidants. However, along with the generation of ozone, excessive ozone will strongly irritate the human respiratory tract, causing symptoms such as sore throat, chest tightness and cough. The hot air in the drying air duct 4 decomposes the ozone by heat, which can effectively reduce the ozone concentration in the circulating air flow.

As shown in Figures 14 to 18, this embodiment introduces a clothing treatment equipment with a drying function, including a clothing treatment drum 2; a drying air duct 4, which is used to supply airflow to the clothing treatment drum 2. The illumination cavity 44 is provided in the drying air duct 4, and at least part of the airflow enters the clothing treatment drum 2 through the illumination cavity 44; an optical plasma tube 3 is provided in the illumination cavity 44 for irradiating and sterilizing the airflow flowing into the clothing treatment drum 2.

In this embodiment, the drying air duct 4 is composed of a clamping space of two vertical plates at the rear of the clothing treatment equipment; the two vertical plates are respectively a back plate 102 and a back cover 103. A gasket 104 made of rubber material is also provided between the back plate 102 and the back cover 103 to seal the drying air duct formed by the two. A retaining rib is provided in the gap space between the back plate 102 and the back cover 103 to separate the gap space between the two plates to form a drying air duct 4 arranged vertically up and down. The lower part of the drying air duct 4 is an air inlet chamber 40, the upper part is an air outlet chamber 42, and the middle part is the connecting cavity 41. At the same time, an air inlet 48 is provided on the back plate 102 on the front side of the lower part of the drying air duct 4, which is used to send the external air into the drying air duct 4 through the fan 9. In addition, a plurality of air outlets 43 are provided on the back plate 102 on the front side of the upper part of the drying air duct 4, which are used to send the airflow of the air outlet chamber 42 into the clothing treatment drum 2.

As shown in Figures 15 to 18, in this embodiment, the drying air duct 4 has a disc-shaped air outlet chamber 42, and a radially extending separation rib 45 is provided in the annular air outlet chamber 42, and the separation rib 45 divides the disc-shaped air outlet chamber 42 into two fan-shaped areas. The first part 421 constitutes the illumination cavity 44, and an optical plasma tube 3 radially inserted into the illumination cavity 44 is provided on the outer peripheral side wall of the disc-shaped air outlet chamber 42.

Preferably, the circumferential size of the first part 421 is much smaller than the circumferential size of the second part 422, so that the optical plasma tube 3 only illuminates the first part 421 of smaller size to enhance the illumination effect of the airflow flowing through the first part 421.

As shown in Figures 14 to 18, in this embodiment, an annular separation rib 453 is provided in the middle of the disc-shaped air outlet chamber 42, and the outer portion of the annular separation rib 453 is provided with a first separation rib 451 and a second separation rib 452 arranged at intervals of angles, and the fan-shaped portion between the first separation rib 451 and the second separation rib 452 constitutes the illumination cavity 44. That is, the first separation rib 451, the second separation rib 452, the annular separation rib 453 and the outer peripheral side wall of the air outlet chamber 42 together enclose two fan-shaped areas of different sizes, namely the first part 421 and the second part 422, wherein the smaller first part 421 is the illumination cavity 44.

In this embodiment, a notch 46 is provided at the first separation rib 451, which is connected to the air inlet chamber 40 through the connecting cavity 41, so that the air inlet of the illumination cavity 44 is located on the side of the first separation rib 451. At the same time, in order to improve the illumination efficiency in the illumination cavity 44, the optical plasma tube 3 is arranged close to the second separation rib 452, so that the airflow flowing into the illumination cavity 44 can be fully illuminated by the optical plasma tube 3, thereby improving the illumination effect.

In this embodiment, the drying air duct 4 also includes an air inlet chamber 40 provided with a fan 9, and the air inlet chamber 40 is directly connected to the second part 422 of the air outlet chamber 42 through the connecting cavity 41 extending obliquely upward. The first separation rib 451 separates the connecting cavity 41 from the illumination cavity 44. The first separation rib 451 is provided with a notch 46 connecting the two sides, and at least part of the airflow flowing into the air inlet chamber 40 flows into the illumination cavity 44 through the notch 46, so as to connect the air inlet chamber 40 with the first part 421 branch of the air outlet chamber 42 through the notch 46. The notch 46 is arranged near the center of the annular air outlet chamber 42 relative to the optical plasma tube 3.

Preferably, as shown in Figures 14 to 18, in this embodiment, the first separation rib 451 extends to the air inlet chamber 40 along the approximate tangential direction of the annular separation rib 453 to form the connecting cavity 41 extending along the common tangent line of the air inlet chamber 40 and the air outlet chamber 42.

In this embodiment, the heater 5 is provided in the second part 422 of the annular air outlet chamber 42. The heater 5 is directly opposite to the air outlet end of the connecting cavity 41 and is used to directly heat the blown-in airflow. At the same time, the heater 5 is arranged close to the notch 46 and can be used to heat the airflow flowing into the illumination cavity 44 to increase the temperature of the airflow flowing through the illumination cavity 44.

As shown in Figures 14 to 18, in this embodiment, the second separation rib 452 extends along a wave line that bends to both sides and reaches the outer periphery of the annular air outlet chamber 42. In this embodiment, the second separation rib 452 is provided with two bending portions 47 that protrude outward and are recessed inward respectively near the outer periphery of the annular air outlet chamber 42, and the columnar optical plasma tube 3 is arranged in the area surrounded by the bending portions 47. By arranging the optical plasma tube 3 in the bending portions 47, the bending portions 47 can guide the airflow in the illumination cavity 44 to form a vortex near the optical plasma tube 3, prolonging the residence time of the airflow there, and further improving the illumination effect.

As shown in Figures 14 to 20, in this embodiment, a plurality of air outlets 43 are provided on one side of the annular air outlet chamber 42, and each air outlet 43 is used to guide the airflow in the air outlet chamber 42 into the clothing treatment drum 2. At least one air outlet 43 of the annular air outlet chamber 42 is a first air outlet 431 connected to the illumination cavity 44, and the remaining air outlets 43 are second air outlets 432 connected to the second part 422. The first air outlet 431 is opened opposite to at least part of the columnar optical plasma tube 3 in the illumination cavity 44. Thus, the columnar optical plasma tube 3 can irradiate the first air outlet 431 area, so that the airflow flowing into the clothing treatment drum 2 can be fully irradiated by the optical plasma tube 3, so as to increase the amount of gas in the clothing treatment drum 2 after illumination, thereby achieving the purpose of improving the sterilization and deodorization effects.

In this embodiment, the first air outlet 431 is located on the offset side of the notch, closer to the center of the air outlet chamber 42 relative to the notch. At the same time, the first air outlet 431 and the notch 46 are both on the same side of the plasma tube 3 to enhance the irradiation effect of the plasma tube on the airflow flowing through the illumination cavity 44.

Preferably, the first air outlet 431 can be arranged at the overlapping position of the columnar optical plasma tube 3, so that the air outlet of the illumination cavity 44 is located at the farthest side relative to the air inlet, so as to extend the flow distance of the airflow in the illumination cavity 44 and improve the illumination effect. At the same time, the second air outlet 432 is opened at the optical plasma tube 3, so that the airflow flowing into the clothing treatment drum 2 can be completely irradiated by the optical plasma tube 3, so as to further increase the amount of gas in the clothing treatment drum 2 after illumination, thereby achieving the purpose of improving the sterilization and deodorization effects (not indicated in the drawings).

In this embodiment, the first air outlet 431 is a circular opening corresponding to one side of the area surrounded by the bending portion 47; the cross-sectional area of the circular opening is smaller than that of the second air outlet 432; the radial dimension of the first air outlet 431 formed by the circular opening is less than or equal to the width of the notch 46, and is approximately equal to the extension length of the optical plasma tube 3 in the illumination cavity 44.

As shown in Figures 14 to 20, in this embodiment, the optical plasma tube 3 is columnar, and the end of the columnar optical plasma tube 3 is provided with a mounting seat 321 located outside the drying air duct 4 and at least partially radially protruding, and the radially protruding portion of the mounting seat 321 is fixed to the outer wall of the drying air duct 4. In this embodiment, radially opposite sides of the mounting seat 321 are respectively provided with fixing ribs 323 protruding radially outward, and both fixing ribs 323 are relatively fitted with the outer wall of the drying air duct 4, and the fixing ribs 323 are respectively provided with through holes, and screws pass through the through holes and are fixed to the drying air duct 4, so as to fix the mounting seat 321 to the drying air duct 4.

Preferably, the outer wall of the drying air duct 4 is provided with a mounting groove, and the bottom of the mounting groove is provided with a through hole for the columnar optical plasma tube 3 to pass through. And the mounting seat has a limiting portion which radially protrudes from the through hole and contacts with the corresponding inner wall of the mounting groove, and the outer periphery of the limiting portion is provided with a shock-absorbing gasket 322 made of elastic material, and the shock-absorbing gasket 322 is clamped between the fixing seat 321 and the outer wall of the drying air duct 4.

The shock-absorbing gasket 322 is cylindrical and coaxially passes through the through hole on the side wall of the drying air duct 4, and the optical plasma tube 3 is located inside the shock-absorbing gasket 322. The outer wall of the shock-absorbing gasket 4 is provided with a circle of notch grooves 324, which are relatively plugged into the side wall of the drying air duct 4 outside the through hole to achieve axial fixed installation between the shock-absorbing gasket 322 and the drying air duct 4. The outer wall of the mounting seat 321 is provided with a circle of protruding ribs 326, which are correspondingly inserted into the annular limiting grooves 325 provided on the inner wall of the cylindrical shock-absorbing gasket 322 to achieve axial fixed installation between the optical plasma tube 3 and the shock-absorbing gasket 322.

In this embodiment, the optical plasma tube 3 may be an ultraviolet light tube having at least two wavelength bands, UVC ultraviolet and UVD ultraviolet.

Ultraviolet rays can be divided into vacuum ultraviolet rays (ultra -low frequency, UVD), short-wave sterilizing ultraviolet rays (low frequency, UVC), medium-wave erythema effect ultraviolet rays (medium frequency, UVB), and long-wave melanoma effect ultraviolet rays (high frequency, UVA) according to their wave lengths. Among them, the UVC band of ultraviolet rays has a wavelength of 200-275nm, and the 253.7nm UVC band ultraviolet rays have a high efficiency in sterilization; the UVD band of ultraviolet rays has a wavelength of 100-200nm, and the 185nm UVD ultraviolet rays can excite oxygen and water in the air to produce optical plasma groups.

In this embodiment, a control device 10 is also included, and the control device 10 is connected to the optical plasma tube 3 to control the optical plasma tube 3 to irradiate the airflow in the drying air duct 4. The control device 10 can be independently installed on the outer wall of the drying air duct 4; it can also be integrated on the control panel of the clothing treatment equipment.

In this embodiment, an optical plasma concentration detection device is provided in the drying air duct 4 or in the clothing treatment drum 2. The optical plasma concentration detection device is used to detect whether the optical plasma concentration in the drying air duct 4 or the optical plasma concentration inside the clothing treatment drum 2 into which sterilizing air is introduced meets the sterilization requirements. The optical plasma concentration detection device is a concentration sensor (not indicated in the drawings).

In this embodiment, an odor detection device is provided inside the clothing treatment drum 2, and the odor detection device is used to detect the odor concentration and taste concentration of the clothing treatment drum 2 or the laundry inside thereof, so as to accurately control the operation of the optical plasma generating module.

In this embodiment, the optical plasma concentration detection device and the odor detection device are respectively connected to the control device 10. The control device 10 receives the optical plasma concentration detected by the optical plasma concentration detection device and the odor concentration and taste concentration values detected by the odor detection device. The control device 10 controls the working time of the optical plasma tube 3 based on the acquired optical plasma concentration, odor concentration and taste concentration values to ensure that the optical plasma concentration in the drying air duct 4 meets the requirements of sterilization and deodorization.

Through the above-mentioned clothing treatment equipment, the optical plasma tube 3 has UVC ultraviolet rays and/or UVD ultraviolet rays. The UVD band ultraviolet rays can efficiently excite oxygen and water in the air to produce optical plasma groups, and the UVC band ultraviolet rays have a high efficiency in sterilization, so that the microorganisms attached to the clothing treatment drum 2 and the clothes therein are completely killed.

In this embodiment, the optical plasma tube 3 may also be a wide-amplitude photon tube, which emits light with balanced energy in a specific wavelength band, with a wavelength of 100nm-300nm.

In this embodiment, a photocatalytic layer is provided in the drying air duct 4, and the wide-amplitude photon tube is provided on one side of the photocatalytic net, or a photocatalytic layer is provided inside the wide-amplitude photon tube, and the photocatalytic layer is composed of nano-scale precious metal catalytic materials. Under the catalysis of specific nano-scale multiple precious metal media, the wide-amplitude photon tube irradiates the air to generate a large amount of hydroxide ions, super oxygen ions, hydrogen peroxide and pure negative oxygen ions to form photohydrogen ions, which can quickly and effectively kill more than 99% of bacteria, viruses and molds in the air, and eliminate odors in the air, thereby achieving the effect of purifying the air.

In this embodiment, the wide-bandwidth photon tube can be a broadband ultraviolet light tube. Compared with the ultraviolet light tube with UVC ultraviolet and/or UVD ultraviolet bands irradiating the air, under the catalysis of specific nano-level multiple precious metal media, the broadband ultraviolet light tube irradiates the air, which can produce a large amount of hydroxide ions, super oxygen ions, hydrogen peroxide and pure negative oxygen ions, thereby improving the sterilization effect.

### Embodiment 4

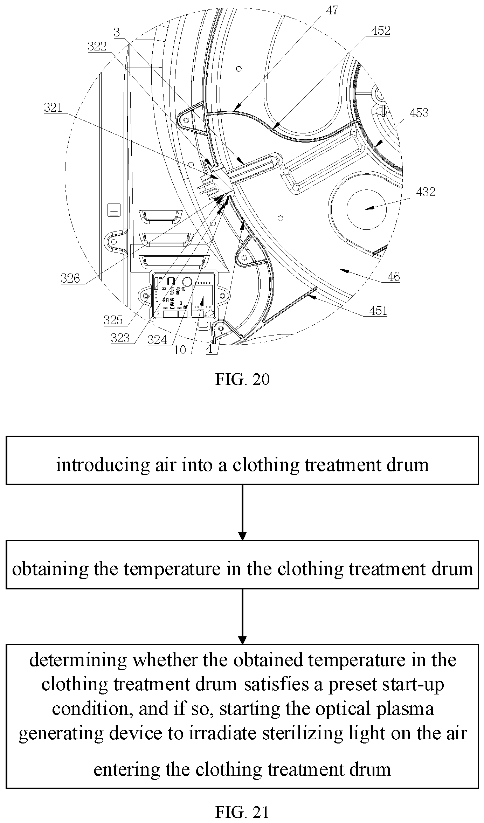

As shown in Figure 21 to Figure 24, the present invention provides a method for controlling a clothing treatment device, comprising: step S1: introducing air into a clothing treatment drum 100; step S2: obtaining the temperature in the clothing treatment drum 100; step S3: determining whether the obtained temperature in the clothing treatment drum 100 satisfies a preset start-up condition, and if so, starting the optical plasma generating device 300 to irradiate sterilizing light on the air entering the clothing treatment drum 100. Through the above-mentioned method for controlling the clothing treatment device, the operation of the optical plasma generating device 300 is controlled according to the temperature in the clothing treatment drum 100, so as to avoid the high temperature in the clothing treatment drum 100 causing the high temperature decomposition of the strong oxidizing substances generated after irradiation, and at the same time, controlling the temperature in the clothing treatment drum 100 is conducive to the volatilization of odorous substances on the clothes, thereby improving the deodorization and sterilization efficiency of the clothes treatment device.

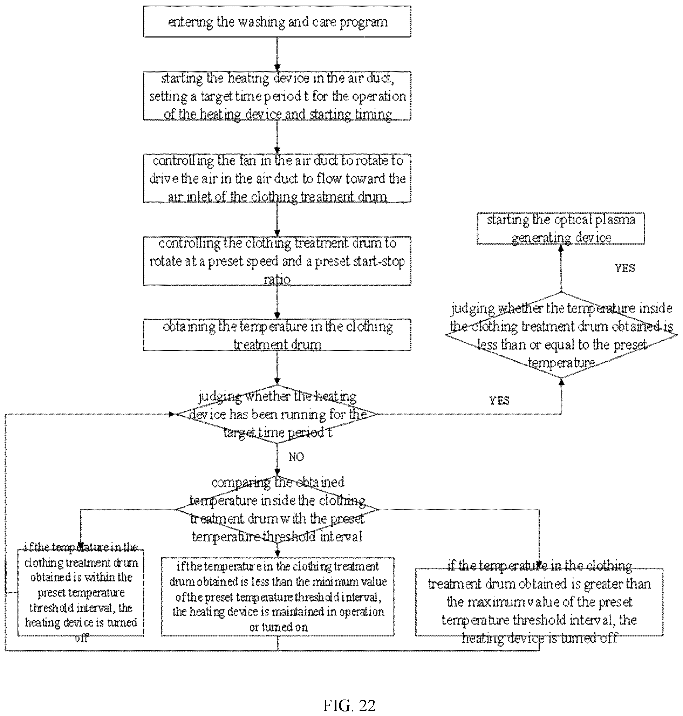

As shown in Figure 22, a method for controlling a clothing treatment equipment is provided in an embodiment of the present invention, step S 1, before air is introduced into the clothing treatment drum 100, it also includes: step S01: entering the washing and care program; step S02: starting the heating device 800 in the air duct 200; step S03: controlling the fan 700 in the air duct 200 to rotate to drive the air in the air duct 200 to flow toward the air inlet of the clothing treatment drum 100.

In this embodiment, the heating device 800 heats the air in the air duct 200 to form hot air. The hot air flowing into the clothing treatment drum 100 is conducive to the volatilization of odorous substances attached to the clothes, especially formaldehyde substances often contained in new clothes generally volatilize quickly at 20 °C ~ 60 °C. At the same time, as the temperature rises, the Brownian motion of air molecules in the air accelerates, which can accelerate the contact between the gas molecules and the oxidizing active substances formed after the optical plasma generating device 300 irradiates the air, thereby accelerating the reaction process.

In this embodiment, whether to start the optical plasma generator 300 is determined according to the outlet air temperature, which is conducive to the strong oxidizing active substances formed after the optical plasma generator 300 irradiates the air to decompose the odor substances, avoiding the high-temperature decomposition of the strong oxidizing active substances, thereby improving the deodorization and sterilization efficiency of the clothing treatment equipment. Optical plasma is a gas containing ions and free electrons generated by nano-light tubes. Optical plasma and ion clusters decompose oxygen and water molecules into hydroxides, free oxygen atoms, superoxide ions and other oxidants. These molecules are extremely unstable and will decompose harmful impurities in the air, turning harmful substances into inert compounds such as carbon dioxide and water, while killing microorganisms, thereby playing a role in sterilization and deodorization.

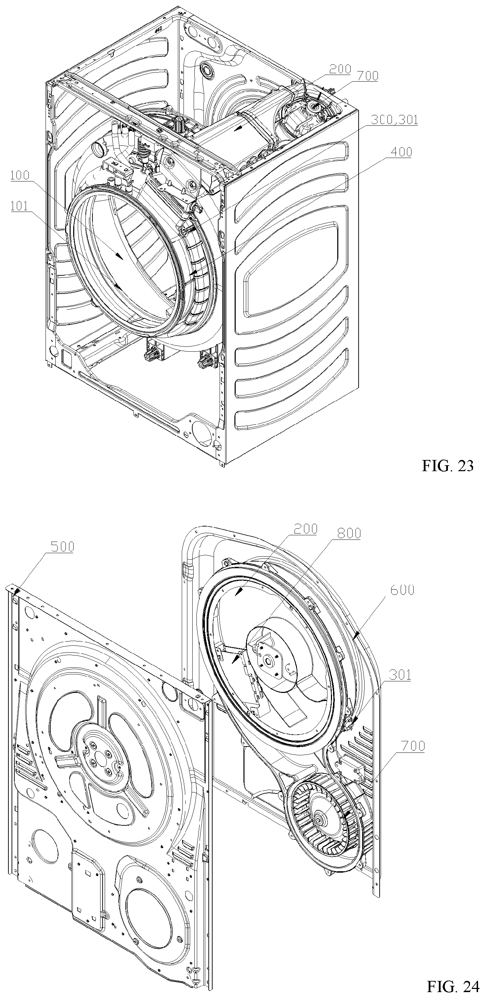

As shown in Figure 23 and Figure 24, in this embodiment, the clothing treatment equipment includes an optical plasma generator 300 and an air duct 200 connected to the clothing treatment drum 100, and the optical plasma generator 300 is used to irradiate the gas flowing into the clothing treatment drum 100 through the air duct 200.

As shown in Figure 22, in this embodiment, step S3, the judgment of whether the temperature inside the clothing treatment drum 100 obtained satisfies the preset start-up condition includes: judging whether the temperature inside the clothing treatment drum 100 obtained is less than or equal to the preset temperature, if the temperature inside the clothing treatment drum 100 obtained is less than or equal to the preset temperature, then starting the optical plasma generating device 300.

In this embodiment, when the temperature in the clothing treatment drum 100 is less than or equal to a preset temperature, the optical plasma generating device 300 is started to irradiate the air flowing into the clothing treatment drum 100 through the air duct 200 to form hydroxide, free oxygen atoms, superoxide ions and other oxidants, thereby preventing the strong oxidizing active substances from being decomposed by heat, thereby utilizing the strong oxidizing active substances to sterilize and deodorize the clothing treatment drum 100 and the clothes therein.

As shown in Figure 22, in this embodiment, step S02, while starting the heating device 800 in the air duct 200, also includes: step S02a: setting a target time period t for the operation of the heating device 800 and starting timing.

In this embodiment, by controlling the target time period of operation of the heating device 800, the clothing treatment drum 100 and the clothing therein are heated, so that the odorous substances attached to the clothing are volatilized faster, and then the odorous substances come into contact with the strong oxidizing substances to accelerate the reaction process.

As shown in Figure 22, in this embodiment, after the step S03, controlling the fan 700 in the air duct 200 to rotate, the method further includes: step S03a: controlling the clothing treatment drum 100 to rotate at a preset speed and a preset start-stop ratio.

In this embodiment, the clothing treatment drum 100 is controlled to rotate at a preset speed and a preset rotation-stop ratio, so that the clothes inside the clothing treatment drum 100 are heated more evenly, thereby promoting the full volatilization of odor substances. During the heating process, the clothing treatment drum 100 is controlled to run at a low speed, with a set speed of 50 rpm and a rotation-stop ratio of 30:5. Preferably, the temperature inside the clothing treatment drum 100 is the outlet temperature of the air outlet of the clothing treatment drum 100. In the process of the air flow flowing from the air inlet to the air outlet of the clothing treatment drum 100, the temperature inside the clothing treatment drum 100 is more stable, and the outlet temperature reflects the overall temperature change inside the clothing treatment drum 100. Through the above-mentioned clothes treatment equipment control method, the heating device 800 heats the air, which is conducive to the volatilization of odor substances attached to the clothes, and whether to start the optical plasma generator 300 is determined according to the outlet temperature, which is conducive to the strong oxidizing active substances formed after the optical plasma generator 300 irradiates the gas to decompose the odor substances, avoiding the high-temperature decomposition of the strong oxidizing active substances, thereby improving the deodorization and sterilization efficiency of the clothing treatment equipment.

As shown in Figures 21 to 24, in this embodiment, step S3, while obtaining the temperature inside the clothing treatment drum 100, also includes: step S3a, judging whether the heating device 800 has been running for the target time period t, if so, turning off the heating device 800; if not, comparing the obtained temperature inside the clothing treatment drum 100 with the preset temperature threshold interval, and controlling the heating device 800 to turn on or off according to the comparison result.

As shown in Figure 22, step S3a, the heating device 800 is controlled to be turned on or off according to the comparison result, specifically including: step S3a1: if the temperature in the clothing treatment drum 100 obtained is within the preset temperature threshold interval, the heating device 800 is turned off; step S3a2: if the temperature in the clothing treatment drum 100 obtained is greater than the maximum value of the preset temperature threshold interval, the heating device 800 is turned off; step S3a3: if the temperature in the clothing treatment drum 100 obtained is less than the minimum value of the preset temperature threshold interval, the heating device 800 is maintained in operation or turned on.

In the present embodiment, when the temperature in the clothing treatment drum 100 is greater than the maximum value of the preset temperature threshold interval, the heating device 800 needs to be turned off to prevent deformation of clothing made of materials that are not resistant to high temperatures, such as silk and wool. When the temperature in the clothing treatment drum 100 is less than the minimum value of the preset temperature threshold interval, the odorous substances attached to the clothing cannot be evaporated quickly to form gaseous molecules, thereby affecting the deodorization efficiency of the clothing treatment equipment.

In this embodiment, after entering the washing and care program, the heating device 800 is started to heat the air in the air duct 200, and the temperature inside the clothing treatment drum 100 is increased from room temperature to within the range of 30 °C to 35 °C, thereby improving the volatilization efficiency of odorous substances. At the same time, after turning off the heating device 800 in the air duct 200, the temperature inside the clothing treatment drum 100 can be reduced back to below 30 °C, thereby avoiding affecting the decomposition of odorous substances by strong oxidizing active substances.

In this embodiment, the preset start condition is that the temperature in the clothing treatment drum is less than or equal to the maximum value of the preset temperature threshold interval, that is, the preset temperature is less than or equal to the maximum value of the preset temperature threshold interval to ensure the sterilization efficiency of the clothing treatment equipment.

As shown in Figure 23, an embodiment of the present invention also provides a clothing treatment equipment using the above-mentioned control method, including: a clothing treatment drum 100, wherein the clothing treatment drum 100 is provided with an air inlet and an air outlet; an air duct 200, wherein a fan 700 and a heating device 800 are provided in the air duct 200, and the air outlet end of the air duct 200 is connected with the air inlet so that the air in the air duct 200 flows into the interior of the clothing treatment drum 100; and an optical plasma generating device 300, wherein the optical plasma generating device 300 is used to irradiate the air flowing into the clothing treatment drum 100 through the air duct 200.

In this embodiment, the hot air blown into the clothing treatment drum 100 is conducive to the volatilization of odorous substances on the clothes, and the optical plasma generating device 300 irradiates the air flowing into the clothing treatment drum 100 through the air duct 200, thereby strengthening the oxidizing substances and improving the deodorization and sterilization efficiency of the clothing treatment equipment.

As shown in Figure 23, in this embodiment, a clothing feeding port 101 is provided at the front end of the clothing treatment drum 100, and a window pad 400 is provided in the clothing feeding port 101, and the top of the window pad 400 is connected to the air outlet end of the air duct 200. The optical plasma generating device 300 includes an optical plasma tube assembly 301, and the optical plasma tube assembly 301 is installed on a side of the window pad 400 close to the air inlet or on a side of the air duct 200 close to the air outlet end of the air duct 200.

In this embodiment, the clothing treatment equipment can be a washer-dryer, and the optical plasma tube assembly 301 is installed on the side of the window pad 400 close to the air inlet or on the air duct 200, so that the oxidizing active substances formed after the optical plasma generating device 300 irradiates the gas can quickly flow into the interior of the clothing treatment drum 100 through the air inlet of the clothing treatment drum 100, thereby reducing the attenuation rate of the strong oxidizing active substances.

As shown in Figures 24, in this embodiment, the clothing treatment equipment also includes a back plate 500 and a back cover 600 arranged on the rear side of the back plate 500, the clothing treatment drum 100 is rotatably supported on the back plate 500, the back cover 600 and the back plate 500 surround an air duct 200 connected to the air inlet, and the optical plasma generating device 300 includes an optical plasma tube assembly 301, and the optical plasma tube assembly 301 is installed in the air duct 200.

In this embodiment, the clothing treatment equipment can be a dryer, and the fan 700 is a drying fan 700. The dryer drives the air flow through the drying fan 700. The drying fan 700 has a large air volume. When driven by the drying fan 700, the odorous air in the drum is blown around the plasma tube. The special wavelength of light released by the plasma tube can make the air produce ion plasma, which can purify the air delivered by the fan 700, and finally blow the purified air into the drum to achieve the sterilization and deodorization effect on the drum and clothes.

The above is only a preferred embodiment of the present invention, and does not limit the present invention in any form. Although the present invention has been disclosed as a preferred embodiment, it is not used to limit the present invention. Any technician familiar with this patent can make some changes or modify the technical contents suggested above into equivalent embodiments without departing from the scope of the technical solution of the present invention. However, any simple modification, equivalent change and modification made to the above embodiments according to the technical essence of the present invention without departing from the content of the technical solution of the present invention still fall within the scope of the solution of the present invention.

## Claims

1. An optical plasma module, comprising: a box body, being provided with an air inlet chamber and an air outlet chamber connected to each other; **characterized in that**,
a fan is installed in the air inlet chamber to draw external air flow from an air inlet at a lower side of the air inlet chamber;
an optical plasma tube is installed in the air outlet chamber, an upper part of the air outlet chamber is communicated to the air inlet chamber, and a lower part of the air outlet chamber is communicated to outside through an air outlet.

2. The optical plasma module according to claim 1, **characterized in that**, one end of an upper chamber of the air outlet chamber is communicated to an outlet of the fan in the air inlet chamber through a passage;
a middle chamber of the air outlet chamber is equipped with the optical plasma tube horizontally inserted from one side of the middle chamber;
a bottom wall of a lower chamber of the air outlet chamber is provided with the air outlet.

3. The optical plasma module according to claim 2, **characterized in that**, the lower chamber of the air outlet chamber is an inverted cone or an inverted frustum with a radial dimension gradually narrowing from top to bottom, and the air outlet is provided at a lowest point of the inverted cone or inverted frustum;
preferably, the lower chamber of the air outlet chamber is an inverted isosceles triangle with left and right sides gradually tilted toward a center, and the air outlet is provided at a lowest point of the inverted isosceles triangle.

4. The optical plasma module according to claim 2, **characterized in that**, the optical plasma tube is coaxially arranged with the air outlet chamber, and axial opposite sides of the air outlet chamber are respectively connected with the air inlet chamber through the passage for an insertion of the optical plasma tube.

5. The optical plasma module according to any one of claims 1 to 4, **characterized in that**, the fan is horizontally arranged in the air inlet chamber, an inlet of the fan is centrally opened downward, an air inlet of the air inlet chamber is arranged at a bottom of the air inlet chamber and is coaxially opened opposite to the inlet of the fan;
the outlet of the fan extends horizontally along a tangential direction of the fan and is connected to the upper chamber of the air outlet chamber through the passage horizontally extended;
preferably, the passage is coaxially arranged with the air outlet chamber.

6. The optical plasma module according to any one of claims 1 to 5, **characterized in that**, the box body comprises a slot body and a top cover, the top cover is buckled and fixedly mounted on a top of the slot body to form the air inlet chamber and the air outlet chamber connected through the passage;
the fan is mounted on the top cover, the optical plasma tube is correspondingly inserted into the air outlet chamber from one side of the slot body and fixedly mounted on the slot body;
preferably, a driving plate is mounted on an outer wall of the slot body.

7. A clothing treatment equipment, comprising: a housing; and a clothing treatment drum installed in the housing; **characterized in that**, the housing is provided with the optical plasma module as claimed in any one of claims 1 to 6, and the air inlet and the air outlet of the optical plasma module are respectively communicated to the clothing treatment drum through pipelines.

8. The clothing treatment equipment according to claim 7, **characterized in that** the box body of the optical plasma module is fixedly installed on the housing, and the optical plasma module is located at a lower side of a top surface of the housing and above the clothing treatment drum.

9. The clothing treatment equipment according to claim 8, **characterized in that**, the housing comprises a front upper crossbeam located at a front side of a top, the box body of the optical plasma module is fixedly installed on a lower side of the front upper crossbeam; the air inlet chamber and the air outlet chamber of the optical plasma module are arranged left and right in the housing.

10. The clothing treatment equipment according to any one of claims 7 to 9, **characterized in that**, a door seal is connected between a drum opening of the clothing treatment drum and a front plate of the housing, and an air inlet j oint and an air outlet j oint are provided on the door seal, both of which can communicate the clothing treatment drum with outside;
the air inlet of the optical plasma module is communicated to the air inlet joint provided on the door seal through an air inlet pipe; the air outlet of the optical plasma module is communicated to the air outlet joint provided on the door seal through an air outlet pipe.

11. A clothing treatment equipment, comprising a clothing treatment drum; a drying air duct, an air outlet end of the drying air duct is communicated to the clothing treatment drum and is configured to supply airflow into the clothing treatment drum; wherein, an optical plasma tube is provided at the air outlet end of the drying air duct for irradiating and sterilizing the airflow flowing into the clothing treatment drum.

12. The clothing treatment equipment according to claim 11, **characterized in that**, the drum opening of the clothing treatment drum is connected to the housing of the clothing treatment equipment through a door seal, a joint is provided on the door seal, and the air outlet end of the drying air duct is communicated to an inside of the clothing treatment drum through the joint provided on the door seal; the optical plasma tube is provided in the joint to perform light irradiation and sterilization on the airflow flowing into the clothing treatment drum through the joint; the optical plasma tube is installed on the door seal, and/or the air outlet end of the drying air duct, and/or the clothing treatment drum.

13. The clothing treatment equipment according to claim 12, **characterized in that**, the optical plasma tube comprises
a base, fixedly mounted with the door seal, with a hollow interior forming an installation chamber;
an optical plasma tube core for generating sterilizing light, installed in the installation chamber of the base;
a light-transmitting area is provided on the base for the sterilizing light generated by the optical plasma tube core to pass through, and the light-transmitting area is arranged toward the inside of the joint.

14. The clothing treatment equipment according to claim 13, **characterized in that**, the base is columnar, and a notch is arranged on one side wall of the columnar base, the notch communicates inside and outside of the columnar base to form the light-transmitting area; the optical plasma tube core arranged inside the columnar base extends along an axis of the columnar base and at least partially overlaps with the notch.

15. The clothing treatment equipment according to claim 14, **characterized in that**, a glass cover is arranged in the installation chamber of the columnar base, the glass cover is cylindrical and is arranged outside the optical plasma tube core, and the cylindrical glass cover at least covers the notch arranged on the side wall of the columnar base.

16. The clothing treatment equipment according to any one of claims 13 to 15, **characterized in that**, the columnar base of the optical plasma tube is inserted from the air inlet end of the joint and extends to the air outlet end of the j oint; the optical plasma tube is arranged on one side of the joint, and the light-transmitting area arranged on one side of the columnar base of the optical plasma tube is opened toward a central axis direction of the joint.

17. The clothing treatment equipment according to claim 16, **characterized in that**, an axial direction of the optical plasma tube is inclined at a certain angle to the axial direction of the joint, an insertion end of the optical plasma tube is inclined relative to an extension end toward the side away from the joint; and the light-transmitting area provided on one side of the columnar base of the optical plasma tube is entirely within the joint.

18. The clothing treatment equipment according to claim 16, **characterized in that**, one end of the columnar base of the optical plasma tube is closed and located in the joint, and an other end is open and located outside the joint, and a fixing cap is detachably installed on the open end of the columnar base outside the joint, and the fixing cap is configured to fix the optical plasma tube in the installation chamber inside the base.

19. The clothing treatment equipment according to claim 18, **characterized in that**, a radially protruding fixing protrusion is provided on an outer periphery of the columnar base of the optical plasma tube, the fixing protrusion is provided between the light-transmitting area of the columnar base and the fixing cap, the fixing cap and the fixing protrusion are respectively located on inner and outer sides of the door seal, and are configured to fix the optical plasma tube on the door seal.

20. The clothing treatment equipment according to claim 18, **characterized in that**, the fixing cap is annular, an inner circumference of the annular fixing cap is smaller than a radial dimension of the optical plasma tube core, and the optical plasma tube core and the glass cover are limited by the fixing cap and installed in the installation chamber of the columnar base.

21. A clothing treatment equipment with drying function, comprising
a clothing treatment drum; and a drying air duct for delivering airflow to the clothing treatment drum; **characterized in that**,
an illumination cavity is provided in the drying air duct, and at least part of the airflow enters the clothing treatment drum through the illumination cavity; and an optical plasma tube is provided in the illumination cavity for irradiating and sterilizing the airflow flowing into the clothing treatment drum.

22. The clothing treatment equipment with drying function according to claim 21, **characterized in that**, the drying air duct has a disc-shaped air outlet chamber, and a separation rib is arranged in the air outlet chamber, and the separation rib divides the disc-shaped air outlet chamber into two parts; a first part with a smaller volume constitutes the illumination cavity, and the optical plasma tube is arranged at a periphery of the illumination cavity and radially inserted into the illumination cavity.

23. The clothing treatment equipment with drying function according to claim 22, **characterized in that**, an annular separation rib is provided in a middle of the disc-shaped air outlet chamber, an outer portion of the annular separation rib is provided with a first separation rib and a second separation rib arranged at an angle interval, a part between the first separation rib and the second separation rib constitutes the illumination cavity, a notch is provided on the first separation rib for introducing the airflow into the illumination cavity, and the optical plasma tube is arranged close to the second separation rib.

24. The clothing treatment equipment with drying function according to claim 22 or 23, **characterized in that**, the drying air duct comprises an air inlet chamber provided with a fan, the air inlet chamber is directly communicated with a second part of the air outlet chamber through a connecting cavity extending obliquely upward; the first separation rib separates the connecting cavity from the illumination cavity, a notch is provided on the first separation rib for connecting the two cavities, at least part of the airflow flowing into the air inlet chamber flows into the illumination cavity through the notch; the notch is arranged close to a center of the annular air outlet chamber relative to the optical plasma tube.

25. The clothing treatment equipment with drying function according to claim 24, **characterized in that**, a heater is provided in the second part of the annular air outlet chamber, the heater is directly arranged opposite to the air outlet end of the connecting cavity, and is configured to directly heat the airflow; the heater is arranged close to the notch on the first separation rib, and is configured to heat the airflow flowing into the illumination cavity.

26. The clothing treatment equipment with drying function according to any one of claims 21 to 25, **characterized in that** the optical plasma tube is columnar, and an end of the columnar optical plasma tube is provided with a mounting seat located outside the drying air duct and at least partially radially protruding, and the radially protruding portion of the mounting seat is fixed to an outer wall of the drying air duct.

27. The clothing treatment equipment with drying function according to claim 26, **characterized in that**, radially opposite sides of the mounting seat are respectively provided with fixing ribs protruding radially outward, the two fixing ribs are relatively fitted with the outer wall of the drying air duct, and the fixing ribs are respectively provided with through holes, screws pass through the through holes and are fixed to the drying air duct, so as to fix the mounting seat on the drying air duct.

28. The clothing treatment equipment with drying function according to claim 26, **characterized in that**, a shock-absorbing gasket is provided on an outer periphery of the columnar optical plasma tube, and the shock-absorbing gasket is clamped between the fixing seat and the drying air duct;
preferably, the shock-absorbing gasket is cylindrical and coaxially passes through the through hole of the side wall of the drying air duct, and the optical plasma tube is located inside the shock-absorbing gasket; a circle of notches is provided on an outer wall of the shock-absorbing gasket, which is relatively plugged into the side wall of the drying air duct outside the through hole; a circle of protruding ribs is provided on an outer wall of the mounting seat, which is correspondingly inserted into the annular limiting groove provided on an inner wall of the cylindrical shock-absorbing gasket.

29. The clothing treatment equipment with drying function according to any one of claims 22 to 28, **characterized in that**, a plurality of air outlets are provided on one side of the annular air outlet chamber, at least one of the air outlets is a first air outlet communicated to the illumination cavity, and other of the air outlets are second air outlets communicated to the second part; the first air outlet is opened opposite to at least part of the columnar optical plasma tube in the illumination cavity.

30. The clothing treatment equipment with drying function according to claim 29, **characterized in that**, the second separation rib is provided with a bending portion protruding outward near an outer periphery of the annular air outlet chamber, and the columnar optical plasma tube is arranged in an area surrounded by the bending portion; the first air outlet is a circular opening corresponding to one side of the area surrounded by the bending portion.

31. A method for controlling a clothing treatment device, **characterized in that** it comprising:
introducing air into a clothing treatment drum;
obtaining a temperature inside the clothing treatment drum;
determining whether the obtained temperature inside the clothing treatment drum satisfies a preset start-up condition, and if so, starting an optical plasma generating device to irradiate the incoming air flowing into the clothing treatment drum with sterilizing light.

32. The method for controlling the clothing treatment equipment according to claim 31, **characterized in that** before the air is introduced into the clothing treatment drum, it also includes: entering a washing and care program; starting a heating device in the air duct; controlling a rotation of the fan in the air duct to drive the air in the air duct to flow toward an air inlet of the clothing treatment drum.

33. The method for controlling the clothing treatment equipment according to claim 31 or 32, **characterized in that** the determination of whether the temperature inside the clothing treatment drum obtained satisfies the preset start-up condition comprises: determining whether the temperature inside the clothing treatment drum obtained is less than or equal to a preset temperature, and if the temperature inside the clothing treatment drum obtained is less than or equal to the preset temperature, starting the optical plasma generating device.

34. The method for controlling the clothing treatment equipment according to claim 32, **characterized in that** the starting of the heating device in the air duct also includes: setting a target time period t for an operation of the heating device and starting timing.

35. The method for controlling the clothing treatment equipment according to claim 34, **characterized in that**, while obtaining the temperature inside the clothing treatment drum, it also includes: determining whether the heating device has been running for a target time period t, and if so, turning off the heating device; if not, comparing the obtained temperature inside the clothing treatment drum with a preset temperature threshold interval, and controlling the heating device to be turned on or off according to the comparison result.

36. The method for controlling the clothing treatment equipment according to claim 35, **characterized in that** the controlling the heating device to be turned on or off according to the comparison result specifically includes: if the temperature in the clothing treatment drum obtained is within the preset temperature threshold interval, turning off the heating device; if the temperature in the clothing treatment drum obtained is greater than a maximum value of the preset temperature threshold interval, turning off the heating device; if the temperature in the clothing treatment drum obtained is less than a minimum value of the preset temperature threshold interval, maintaining operation or turning on the heating device.

37. The method for controlling the clothing treatment equipment according to claim 35, **characterized in that** the preset start condition is that the temperature in the clothing treatment drum is less than or equal to the maximum value of the preset temperature threshold range.

38. A clothing treatment equipment using the control method described in any one of claims 31 to 37, **characterized in that** it comprises: a clothing treatment drum, the clothing treatment drum is provided with an air inlet and an air outlet; an air duct, a fan and a heating device are provided in the air duct, the air outlet end of the air duct is connected with the air inlet so that the air in the air duct can flow into an interior of the clothing treatment drum; an optical plasma generating device, the optical plasma generating device is configured to irradiate the air flowing into the clothing treatment drum through the air duct.

39. The clothing treatment equipment according to claim 38, **characterized in that**, a clothing feeding port is provided at a front end of the clothing treatment drum, a window pad is provided in the clothing feeding port, and a top of the window pad is connected to the air outlet end of the air duct; the optical plasma generating device includes an optical plasma tube assembly, and the optical plasma tube assembly is installed on a side of the window pad close to the air inlet or on a side of the air duct close to the air outlet end of the air duct.

40. The clothing treatment equipment according to claim 38, **characterized in that**, it includes a back plate and a back cover arranged on a rear side of the back plate, the clothing treatment drum is rotatably supported on the back plate, and the back cover and the back plate surround an air duct communicated to the air inlet; the optical plasma generating device includes the optical plasma tube assembly, and the optical plasma tube assembly is installed in the air duct.
